(19) Europäisches Patentamt
European Patent Office
Office européen des brevets

(11) EP 0 888 341 B1

(12) FASCICULE DE BREVET EUROPEEN

(45) Date de publication et mention de la délivrance du brevet: **30.11.2005 Bulletin 2005/48**

(21) Numéro de dépôt: **97907157.8**

(22) Date de dépôt: **03.03.1997**

(51) Int Cl.[7]: **C07D 405/12**, C07D 333/24, C07D 257/04, C07C 323/60, C07D 209/18, A61K 31/405, A61K 31/195

(86) Numéro de dépôt international: **PCT/FR1997/000367**

(87) Numéro de publication internationale: **WO 1997/032874 (12.09.1997 Gazette 1997/39)**

# (54) NOUVEAUX DERIVES SOUFRES COMPORTANT UNE LIAISON AMIDE, LEUR PROCEDE DE PREPARATION, LEUR APPLICATION A TITRE DE MEDICAMENTS ET LES COMPOSITIONS PHARMACEUTIQUES LES RENFERMANT

EINE AMIDGRUPPE TRAGENDE SCHWEFELHALTIGE VERBINDUNGEN, VERFAHREN ZU IHRER HERSTELLUNG, DEREN VERWENDUNG IN MEDIKAMENTEN UND SIE ENTHALTENDE PHARMAZEUTISCHE ZUBEREITUNGEN

NOVEL SULPHUR DERIVATIVES COMPRISING AN AMIDE BOND, METHOD FOR PREPARING SAME, USE THEREOF AS DRUGS, AND PHARMACEUTICAL COMPOSITIONS CONTAINING SUCH DERIVATIVES

(84) Etats contractants désignés:
**AT BE CH DE DK ES FI FR GB GR IE IT LI LU NL PT SE**

(30) Priorité: **04.03.1996 FR 9602672**

(43) Date de publication de la demande:
**07.01.1999 Bulletin 1999/01**

(73) Titulaires:
- **Aventis Pharma S.A.
92160 Antony (FR)**
- **INSTITUT NATIONAL DE LA SANTE ET DE LA RECHERCHE MEDICALE (INSERM)
75654 Paris Cédex 13 (FR)**

(72) Inventeurs:
- **DEPREZ, Pierre
F-94320 Thiais (FR)**
- **DUMAS, Jacques
F-93360 Neuilly-Plaisance (FR)**
- **FOURNIE-ZALUSKI, Marie-Claude
F-75011 Paris (FR)**
- **GUILLAUME, Jacques
F-93190 Livry-Gargan (FR)**
- **ROQUES, Bernard, Pierre
F-94440 Saint-Maurice (FR)**

(56) Documents cités:
**DE-A- 2 349 707 US-A- 4 053 651**

- **CHEMICAL ABSTRACTS, vol. 122, no. 19, 8 Mai 1995 Columbus, Ohio, US; abstract no. 230128, J.L. BALWIERCZAK ET AL.: "Effects of metalloproteinase inhibitors on smooth muscle endothelin-converting enzyme activity" XP002019777 & BIOCHEM. PHARMACOL., vol. 49, no. 3, 1995, pages 291-296,**
- **JOURNAL OF MEDICINAL CHEMISTRY, vol. 37, no. 8, 1994, WASHINGTON US, pages 1070-1083, XP002019771 M.-C. FOURNI -ZALUSKI ET AL.: "New Dual Inhibitors of Neutral Endopeptidase and Angiotensin-Converting Enzyme:..."**
- **CHEMICAL AND PHARMACEUTICAL BULLETIN, vol. 35, no. 6, 1987, TOKYO JP, pages 2382-2387, XP002019773 T. KOMORI ET AL.: "Sulfur-Containing Acylamino Acids. I. ..."**
- **CHEMICAL AND PHARMACEUTICAL BULLETIN, vol. 35, no. 6, 1987, TOKYO JP, pages 2388-2393, XP002019774 T. KOMORI ET AL.: "Sulfur-Containing Acylamino Acids. II. ..."**
- **CHEMICAL AND PHARMACEUTICAL BULLETIN, vol. 29, no. 1, 1981, TOKYO JP, pages 63-70, XP002019775 M. OYA ET AL.: "Thiol Compounds. I. Synthesis and Antihypertensive Activity of Mercaptoacylamino Acids"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



(Cont. page suivante)

- JOURNAL OF MEDICINAL CHEMISTRY, vol. 35, no. 13, 1992, WASHINGTON US, pages 2473-2481, XP002019768 M.-C. FOURNI -ZALUSKI ET AL.: ""Mixed Inhibitor-Prodrug" as a New Approach toward Systematically Active Inhibitors of Enkephalin-Degrading Enzyme"
- JOURNAL OF MEDICINAL CHEMISTRY, vol. 29, no. 5, 1986, WASHINGTON US, pages 751-757, XP002019769 M.-C. FOURNI -ZALUSKI ET AL.: "1H-NMR Configurational Correlation for Retro-Inverso Dipeptides: Application to the Determination of the Absolute Configuration of the "Enkephalinase" Inhibitors. ...."
- JOURNAL OF MEDICINAL CHEMISTRY, vol. 28, no. 9, 1985, WASHINGTON US, pages 1158-1169, XP002019770 M.-C. FOURNI -ZALUSKI ET AL.: "New Bidentates as Full Inhibitors of Enkephalin-Degrading Enzymes: Synthesis and Analgesic Properties"
- JOURNAL OF MEDICINAL CHEMISTRY, vol. 37, no. 15, 1994, WASHINGTON US, pages 2461-2476, XP002019772 B.R. NEUSTADT ET AL.: "Mercaptoacyl Amino Acid Inhibitors of Atriopeptidase. ..."
- JOURNAL OF ORGANIC CHEMISTRY, vol. 50, no. 11, 1985, EASTON US, pages 1830-1835, XP002019776 D.A. EVANS ET AL.: "Asymmetric Synthesis of the Enkephalinase Inhibitor Thiorphan"
- CHEMICAL ABSTRACTS, vol. 125, no. 5, 29 Juillet 1996 Columbus, Ohio, US; abstract no. 54282, K. BARNES ET AL.: "Metallopeptidase inhibitors induce an up-regulation of endothelin-converting enzyme levels and its redistribution from the plasma membrane to an intracellular compartment" XP002019778 & J. CELL SCI., vol. 109, no. 5, 1996, pages 919-928,

## Description

La présente invention concerne de nouveaux dérivés soufrés comportant une liaison amide, leur procédé de préparation, les nouveaux intermédiaires obtenus, leur application à titre de médicaments, les compositions pharmaceutiques les renfermant et la nouvelle utilisation de tels dérivés.
L'article Biochem. Pharmacol., vol. 49, 1995, pages 291-296 décrit le thiophan.
L'article Journal of Médicinal Chemistry, vol. 37, 1994, pages 1070-83 décrit des inhibiteurs de l'endopeptidase et de l'enzyme de conversion de l'angiotensine.
L'article Journal of Médicinal Chemistry, vol. 35, 1992, pages 2473-81 décrit des inhibiteurs des enzymes de dégradation de l'enképhaline.
Le document DE-A-3 819 539 décrit des inhibiteurs de l'endopeptidase.
La présente invention a pour objet l'utilisation pour la préparation de médicaments destinés au traitement de pathologies requérant une inhibition de l'enzyme de conversion de l'endothéline, ces pathologies étant les spasmes vasculaires, le vasospasme consécutif à une hémorragie cérébrale, les spasmes coronariens, les spasmes vasculaires périphériques, les insuffisances rénales, l'infarctus du myocarde, l'insuffisance cardiaque congestive, l'athérosclérose, l'hypertension pulmonaire, l'asthme, l'ostéoporose, l'hypertrophie prostatique, des produits de formule (I) :

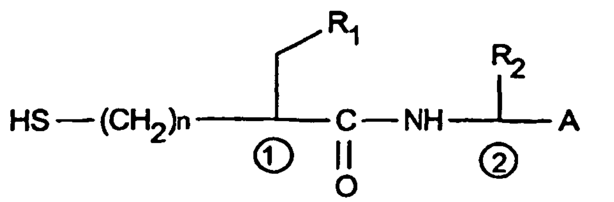

(I)

dans laquelle :

n représente l'entier 0 ou 1,
$R_1$ représente un radical phényle ou biphényle éventuellement substitué par un ou plusieurs radicaux choisis parmi les atomes d'halogène, les radicaux hydroxyle éventuellement protégé, alcoxy linéaire ou ramifié renfermant au plus 4 atomes de carbone, phénoxy, cyano, carboxy libre, salifié, estérifié ou amidifié, benzyloxy et le radical dioxol,
$R_2$ représente un radical méthyle substitué par un radical phényle, phénylthio ou indolyle et éventuellement par un deuxième radical phényle, ces radicaux phényle, phénylthio et indolyle étant éventuellement substitués par un ou plusieurs radicaux choisis parmi les atomes d'halogène, les radicaux hydroxyle éventuellement protégé, alcoxy linéaire ou ramifié renfermant au plus 4 atomes de carbone, cyano, carboxy libre, salifié, estérifié ou amidifié, benzyloxy, thiényle, naphtyle et phényle, ces trois derniers radicaux étant eux-mêmes éventuellement substitués par un ou plusieurs radicaux choisis parmi les atomes d'halogène, les radicaux hydroxyle éventuellement protégé, alcoxy linéaire ou ramifié renfermant au plus 4 atomes de carbone, cyano et carboxy libre, salifié, estérifié ou amidifié,
A représente le radical carboxy libre, salifié, estérifié ou amidifié, le radical tétrazolyle libre ou salifié, ou un radical alkyle, renfermant au plus 10 atomes de carbone et substitué par un radical choisi parmi les radicaux carboxy libre, salifié, estérifié ou amidifié, les radicaux hydroxyle éventuellement protégé, alcoxy renfermant au plus 4 atomes de carbone, phénoxy, phényle, naphtyle, thiényle, indolyle et pyridyle, ces radicaux étant éventuellement substitués par un ou plusieurs radicaux choisis parmi les atomes d'halogène, les radicaux hydroxyle éventuellement protégé, alcoxy linéaire ou ramifié renfermant au plus 4 atomes de carbone, cyano et carboxy libre, salifié, estérifié ou amidifié,
① et ② indiquant les centres, le cas échéant, asymétriques des produits de formule (I),

lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).
Dans les produits de formule (I) et dans ce qui suit :

- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés,
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés,
- le terme atome d'halogène désigne de préférence l'atome de brome, mais peut aussi représenter un atome de fluor, de chlore ou d'iode.

Le ou les radicaux carboxy des produits de formule (I) peuvent être salifiés ou estérifiés par les groupements divers connus de l'homme du métier parmi lesquels on peut citer, par exemple :

- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaine, la lysine, l'arginine, l'histidine, la N-méthylglucamine.
  On préfère les sels de sodium ou de potassium,
- parmi les composés d'estérification, les radicaux alkyle pour former des groupes alcoxy carbonyle ou arylalcoxycarbonyle, tels que, par exemple, méthoxycarbonyle, éthoxycarbonyle, n-propoxy- et isopropoxy-carbonyle, n-butoxy-, isobutoxy- et tert-butoxy-carbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les groupes du type -CON($R_6$) ($R_7$) dans lesquels les radicaux $R_6$ et $R_7$ identiques ou différents représentent un atome d'hydrogène ou un radical alkyle ayant de 1 à 4 atomes de carbone tels que les radicaux méthyle, éthyle, propyle, isopropyle, butyle, isobutyle, sec-butyle ou tert-butyle.

Parmi les groupes -CON($R_6$) ($R_7$) définis ci-dessus, on préfère ceux dans lesquels le radical -N($R_6$) ($R_7$) représente le radical amino, mono ou diméthylamino.

Le radical N($R_6$) ($R_7$) peut également représenter un hétérocycle qui peut ou non comporter un hétéroatome supplémentaire. On peut citer les radicaux pyrrolyle, imidazolyle, indolyle, pipéridino, morpholino, pipérazinyle. On préfère les radicaux pipéridino ou morpholino.

Des exemples de groupement protecteur du radical hydroxyle protégé sont donnés notamment dans le livre usuel de l'homme du métier : Protective Groups in Organic Synthesis, Theodora W. Greene, Harvard University, imprimé en 1981 par Wiley-Interscience Publishers, John Wiley & Sons.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoique, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzène-sulfonique et les acides aryldisulfoniques.

On peut citer plus particulièrement les sels formés avec les acides chlorhydrique ou méthanesulfonique par exemple.

On peut rappeler que la stéréoisomérie peut être définie dans son sens large comme l'isomérie de composés ayant mêmes formules développées, mais dont les différents groupes sont disposés différemment dans l'espace.

Il est entendu que la définition des produits de formule (I) telle que définie ci-dessus comprend tous les stéréoisomères possibles, toutes les modifications racémiques, tous les isomères optiques et tous les mélanges de ces produits qui possèderaient l'activité indiquée ci-après.

Les produits de formule (I) comportent notamment deux centres ① et ② , ① étant asymétrique et ② étant asymétrique lorsque $R_2$ ne représente pas un atome d'hydrogène.

La présente invention a notamment pour objet l'utilisation telle que définie ci-dessus des produits de formule (I) dans laquelle lorsque n représente l'entier 1, le premier centre asymétrique ① est de préférence sous forme S, et lorsque n représente l'entier 0, le premier centre asymétrique ① est de préférence sous forme R, le second centre ② pouvant être de préférence sous forme S.

La présente invention a particulièrement pour objet l'utilisation telle que définie ci-dessus des produits de formule (I) telle que définie ci-dessus, dans laquelle :

n représente l'entier 0 ou 1,

$R_1$ représente un radical phényle ou biphényle éventuellement substitué par un ou deux radicaux choisis parmi les atomes d'halogène, les radicaux hydroxyle éventuellement protégé, alcoxy linéaire ou ramifié renfermant au plus 4 atomes de carbone, benzyloxy et le radical dioxol,

$R_2$ représente un radical méthyle substitué par un radical phényle, phénylthio ou indolyle et éventuellement par un deuxième radical phényle, ces radicaux phényle, phénylthio et indolyle étant éventuellement substitués par un radical choisi parmi les radicaux hydroxyle éventuellement protégé, alcoxy linéaire ou ramifié renfermant au plus 4 atomes de carbone, benzyloxy, thiényle, naphtyle et phényle lui-même éventuellement substitué par un radical hydroxyle éventuellement protégé ou alcoxy linéaire ou ramifié renfermant au plus 4 atomes de carbone,

et A représente le radical carboxy libre, salifié, estérifié ou amidifié, tétrazolyle libre ou salifié, ou un radical alkyle, renfermant au plus 8 atomes de carbone et substitué par un radical choisi parmi les radicaux carboxy libre, salifié, estérifié ou amidifié, les radicaux hydroxyle éventuellement protégé, alcoxy renfermant au plus 4 atomes de carbone, et phénoxy,

lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

La présente invention a plus particulièrement pour objet l'utilisation telle que définie ci-dessus des produits de formule (I) telle que définie ci-dessus, dans laquelle n, $R_2$ et A ont les significations indiquées ci-dessus, et $R_1$ représente un radical phényle ou biphényle, éventuellement substitué soit par un atome de brome ou un radical benzyloxy, soit par un radical dioxol et éventuellement un atome d'halogène,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

La présente invention a encore plus particulièrement pour objet l'utilisation telle que définie ci-dessus des produits de formule (I) telle que définie ci-dessus, dans laquelle :

n représente l'entier 1,

$R_1$ représente un radical phényle ou biphényle, éventuellement substitué soit par un atome de brome ou un radical benzyloxy, soit par un radical dioxol et éventuellement un atome d'halogène,

$R_2$ représente un radical méthyle substitué soit par un radical indolyle lui-même éventuellement substitué par un radical benzyloxy, soit par deux radicaux phényle, soit par un radical phénylthio ou phényle, lui-même éventuellement substitué par un radical thiényle, naphtyle ou phényle lui-même éventuellement substitué par un radical hydroxyle éventuellement protégé, alcoxy renfermant au plus 4 atomes de carbone ou benzyloxy,

A représente le radical carboxy libre, salifié, estérifié ou amidifié ou un radical alkyle, renfermant au plus 8 atomes de carbone substitué par un radical phénoxy,

lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

Notamment, $R_1$ peut représenter un radical phényle ou biphényle, éventuellement substitué par un atome de brome.

La présente invention a tout particulièrement pour objet l'utilisation telle que définie ci-dessus des produits de formule (I) telle que définie ci-dessus, dont les noms suivent :

- N-[3-(3-bromophényl)-2-(mercaptométhyl)-1-oxopropyl]-L-Tryptophane,
- N-(3-((1,1'-biphényl)-3-yl)-2-(mercaptométhyl)-1-oxopropyl]-L-Tryptophane,
- (R,S) N-(3-(4-bromophényl)-2-(mercaptométhyl)-1-oxopropyl] L-Tryptophane,
- (R,S) N-(3-((1,1'-biphényl)-4-yl)-2-(mercaptométhyl)-1-oxopropyl]-L-Tryptophane,
- (S) N-[2-(mercaptométhyl)-1-oxo-3-phénylpropyl]-4-(2-thiényl)-L-Phénylalanine,
- (S) N-[2-(mercaptométhyl)-1-oxo-3-phénylpropyl]-4-(1-naphtyl)-L-Phénylalanine,
- (S) N-(2-(mercaptométhyl) 1-oxo-3-phénylpropyl] L-Tryptophane,
- (S) 4-phényl-N-((2-mercaptométhyl)-1-oxo-3-phénylpropyl]-L-Phénylalanine,
- (S,S) N-(2-((1,1'-biphényl)-4-yl)-1-(1H-tétrazol-5-yl)-éthyl)-alpha-(mercaptométhyl)-benzènepropanamide,
- (R) et (S) N-[3-(3-bromophényl)-2-(mercaptométhyl)-1-oxopropyl]-4-(2-thiényl)-L-Phénylalanine (isomères A et B),
- N-[3-(3-bromophényl)-2-(mercaptométhyl)-1-oxopropyl]-7-(phénylméthoxy)-Tryptophane,

lesdits produits de formule (I) étant sous toutes les formes isomères racémiques, énantiomères et diastéréoisomères

possibles, ainsi que les sels d'addition avec les acides minéraux et organiques ou avec les bases minérales et organiques desdits produits de formule (I).

La présente invention a encore pour objet un procédé de préparation des produits de formule (I), telle que définie ci-dessus, caractérisé en ce que **soit** l'on soumet un produit de formule (II) :

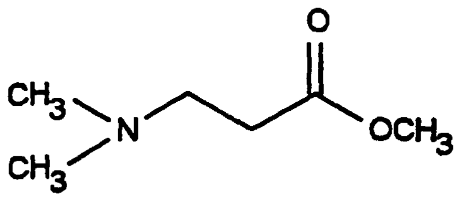

(II)

à l'action d'un produit de formule (III) :

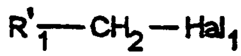

(III)

dans laquelle $Hal_1$ représente un atome de brome ou d'iode, $R'_1$ a la signification indiquée ci-dessus pour $R_1$ dans laquelle les éventuelles fonctions réactives sont éventuellement protégées, pour obtenir le produit de formule (IV) :

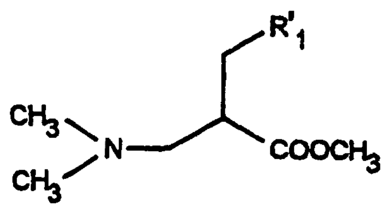

(IV)

dans laquelle $R'_1$ a la signification indiquée ci-dessus, que l'on soumet à l'action de l'iodure de méthyle pour obtenir le produit de formule (V) :

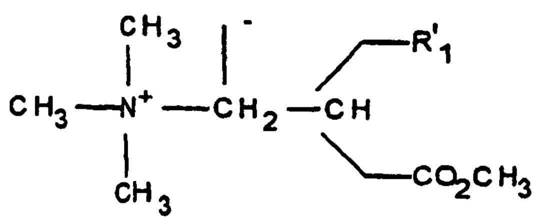

(V)

dans laquelle $R'_1$ a la signification indiquée ci-dessus, que l'on soumet à une réaction d'élimination et de saponification, pour obtenir le produit de formule (VI) :

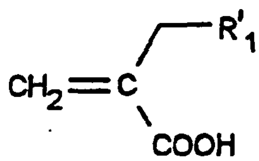

(VI)

dans laquelle $R'_1$ a la signification indiquée ci-dessus pour $R_1$, que l'on soumet à l'action du composé de formule (XIIa) :

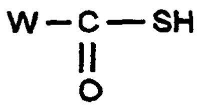

(XIIa)

dans lequel W représente un radical alkyle ou phényle, pour obtenir le produit de formule (VII) :

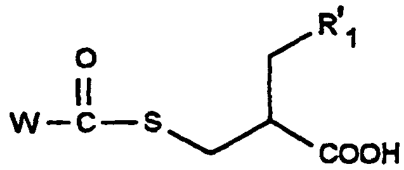

(VII)

dans laquelle $R'_1$ et W ont les significations indiquées ci-dessus, que l'on soumet :

ou bien à l'action, en présence d'un agent couplant, d'un produit de formule (VIII) :

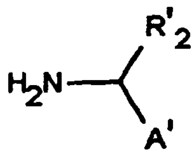

(VIII)

dans laquelle $R'_2$ a la signification indiquée ci-dessus et A' a la signification indiquée ci-dessus pour A, dans laquelle les éventuelles fonctions réactives sont éventuellement protégées, pour obtenir le produit de formule (IX) :

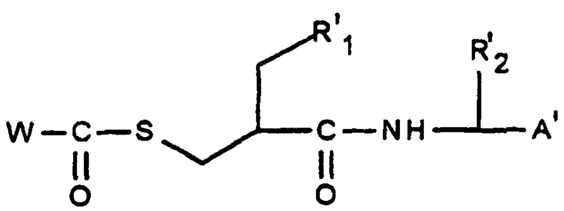

(IX)

dans laquelle $R'_1$, $R'_2$, W et A' ont les significations indiquées ci-dessus,
ou bien à l'action d'un agent chlorant pour obtenir le chlorure d'acide correspondant, que l'on fait réagir avec le composé de formule (VIII) telle que définie ci-dessus, pour obtenir le produit de formule (IX) tel que définie ci-dessus, **soit** l'on soumet un produit de formule (X) :

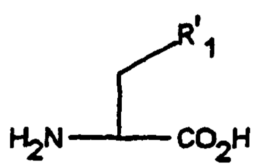

(X)

dans laquelle $R'_1$ a la signification indiquée ci-dessus, à une réaction de diazotation en présence d'un agent halogénant, pour obtenir le produit de formule (XI) :

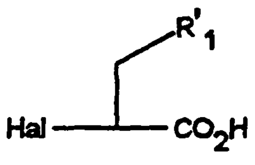

(XI)

dans laquelle $R'_1$ a la signification indiquée ci-dessus et Hal représente un atome d'halogène, que l'on soumet à l'action du thioacétate de formule (XIIb) :

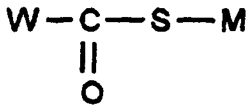

(XIIb)

dans laquelle M représente un métal alcalin et W a la signification indiquée ci-dessus, pour obtenir le produit de formule (XIII) :

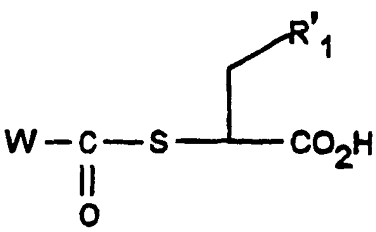

(XIII)

dans laquelle W et $R'_1$ ont les significations indiquées ci-dessus, que l'on soumet à l'action du produit de formule (VIII) telle que définie ci-dessus, pour obtenir le produit de formule (XIV) :

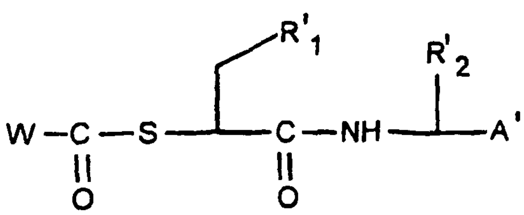

(XIV)

dans laquelle $R'_1$, $R'_2$, A' et W ont les significations indiquées ci-dessus,
produits de formules (IX) et (XIV) que, pour obtenir des produits de formule (I) ou pour transformer des produits de formule (I) en d'autres produits de formule (I), l'on peut traiter, si désiré et si nécessaire, à l'une ou plusieurs des réactions suivantes, dans un ordre quelconque :

- une réaction de saponification de fonction ester en fonction acide,
- une réaction de transformation de fonction cyano en fonction acide ou tétrazolyle,
- une réaction de transformation de fonction alcoxy en fonction hydroxyle,
- une réaction d'estérification, salification ou amidification de fonction acide,
- une réaction de libération de la fonction thiol à partir du radical

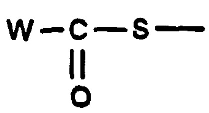

- une réaction d'élimination des groupements protecteurs que peuvent porter les fonctions réactives protégées,
- une réaction de salification par un acide ou une base minéral(e) ou organique pour obtenir le sel correspondant, lesdits produits de formule (I) ainsi obtenus étant sous toutes les formes isomères possibles racémiques, énantiomères et diastéréoisomères.

Dans des conditions préférentielles de mise en oeuvre de l'invention, le procédé décrit ci-dessus peut être réalisé de la façon suivante :

- la réaction du produit de formule (II) telle que définie ci-dessus avec le produit de formule (III) telle que définie ci-dessus est réalisée de préférence en présence d'une base forte telle que par exemple le LDA, LiHMDS, KHMDS, l'hydrure de sodium ou de potassium en présence, si nécessaire, d'un agent chélatant tel que par exemple le DMPU ou encore le HMPA.

On peut noter que dans le produit de formule (II), les radicaux méthyle et notamment celui de l'ester d'acide peuvent être d'autres radicaux alkyle tel que éthyle, propyle ou butyle.

La réaction du produit de formule (IV) avec l'iodure de méthyle est réalisée de préférence dans un solvant polaire tel que alcoolique comme par exemple dans l'isopropanol.

On peut noter que le produit de formule (IV) peut être soumis à diverses réactions connues de l'homme du métier pour transformer le radical $R'_1$ en un autre radical $R'_1$ correspondant à un radical $R_1$, tel que défini par la formule (I) des produits de la présente invention. Une illustration d'une telle transformation de $R'_1$ est donnée dans la préparation de l'exemple 5 décrite ci-après.

Les réactions d'élimination et de saponification auxquelles est soumis le produit de formule (V) s'effectue de préférence au reflux d'une base aqueuse telle que par exemple la soude ou la potasse.

Le produit de formule (VI) ainsi obtenu est soumis à l'action du composé de formule (XIIa) à chaud ou à température ambiante ainsi qu'il est illustré dans les préparations des exemples décrites ci-après.

On peut noter que le composé de formule (VII) ainsi obtenu peut être sous forme racémique ou peut être dédoublé par les méthodes usuelles connues de l'homme du métier telles que notamment sous forme de sel d'éphédrine par exemple par la méthode décrite dans la référence indiquée au stade 3 de l'exemple 10 dont la préparation est décrite ci-après.

L'agent de couplage utilisé pour la réaction du produit de formule (VII) avec le produit de formule (VIII) peut être notamment l'EDC ou le DCC dans le chlorure de méthylène ou encore le BOP dans du cyanure de méthyle ou le dichlorométhane en présence de triéthylamine.

L'agent chlorant auquel peut être soumis le produit de formule (VII) peut être tout agent connu de l'homme du métier tel que par exemple $PCl_5$, $SOCl_2$ ou $POCl_3$.

La réaction de diazotation du produit de formule (X) pour obtenir le produit de formule (XI) peut être réalisée notamment par du nitrite de sodium dans un acide aqueux tel que l'acide sulfurique en présence d'un agent nucléophile tel que par exemple le bromure de potassium. Dans le produit de formule (XI), Hal représente ainsi notamment un atome de brome.

La réaction du produit de formule (XI) avec le produit de formule ($XII_b$) peut être réalisée dans un solvant tel que par exemple le DMF ou le DMA.

Dans le composé de formule (XIIb), M peut notamment représenter un atome de césium ou de potassium.

Dans les composés de formules (XIIa) et (XIIb), W représente notamment le radical méthyle ou phényle.

La réaction du produit de formule (XIII) avec le composé de formule (VIII) pour obtenir le produit de formule (XIV) peut être réalisée comme indiqué ci-dessus pour le composé de formule (VII) et notamment dans un solvant neutre tel que le chlorure de méthylène.

Selon les valeurs de $R'_1$, $R'_2$ et A', les produits de formules (IX) et (XIV) constituent ou non des produits de formule (I) et peuvent donner des produits de formule (I), ou être transformés en d'autres produits de formule (I) en étant soumis à une ou plusieurs des réactions indiquées ci-dessus qui peuvent être réalisées, par exemple, comme indiqué ci-après.

Les diverses fonctions réactives que peuvent porter certains composés des réactions définies ci-dessus peuvent, si nécessaire, être protégées : il s'agit par exemple des radicaux hydroxyle ou carboxy libre qui peuvent être protégés par les groupements protecteurs appropriés.

La liste suivante, non exhaustive, d'exemples de protection de fonctions réactives peut être citée :

- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle.

Les produits décrits ci-dessus peuvent, si désiré, faire l'objet, sur les éventuelles fonctions carboxy, de réactions d'estérification, de salification ou d'amidification, qui peuvent être réalisées selon les méthodes usuelles connues de l'homme du métier.

Les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante.

Les fonctions acide peuvent être protégées par exemple sous forme d'esters formés avec les esters facilement clivables tels que les esters benzyliques ou ter butyliques ou des esters connus dans la chimie des peptides.

Les éventuelles réactions de saponification de fonction ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou par l'hydroxyde de lithium dans le tétrahydrofuranne aqueux ou encore par de l'acide chlorhydrique ou sulfurique.

Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions cyano des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :

J. Organometallic Chemistry., <u>33</u>, 337 (1971) KOZIMA S.& coll.

Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.

Les éventuelles fonctions hydroxyle des produits décrits ci-dessus peuvent être, si désiré, transformées en fonction acide par oxydation dans les conditions usuelles connues de l'homme du métier telles que par exemple par action du réactif de Jones pour accéder aux acides.

L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.

On trouvera une liste de différents groupements protecteurs utilisables par exemple dans le brevet BF 2 499 995.

Les produits décrits ci-dessus peuvent, si désiré, faire l'objet de réactions de salification par exemple par un acide minéral ou organique ou par une base minérale ou organique selon les méthodes usuelles connues de l'homme du métier.

Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les structures et propriétés de l'endothéline et de son précurseur la Big Endothéline sont décrites dans la littérature comme par exemple dans le document WO 93/11154.

Les produits de formule (I) de la présente invention ont été trouvés comme possédant une activité inhibitrice de l'enzyme de conversion de l'endothéline qui permet d'obtenir à partir de la Big Endothéline, l'endothéline proprement dite, qui est donc un agent vasoconstricteur extrêmement puissant.

Les produits de formule (I) de la présente invention peuvent donc être utiles dans le traitement d'affections résultant de quantités anormalement élevées d'endothéline.

Les composés de formule (I) telle que définie ci-dessus ainsi que leurs sels d'addition tels que définis ci-dessus présentent d'intéressantes propriétés pharmacologiques.

Les produits de formule (I) telle que définie ci-dessus, doués de propriétés inhibitrices de l'enzyme de conversion de l'endothéline, peuvent ainsi notamment réduire les quantités et ainsi les effets de l'endothéline, notamment des effets vasoconstricteurs et hypertenseurs induits par l'endothéline. On note en particulier un effet antiischémique.

Les produits de formule (I) ont ainsi également pour effet de réduire les effets stimulants de l'endothéline au

niveau de tous les types cellulaires, notamment les cellules musculaires lisses, les fibroblastes, les cellules neuronales et les cellules osseuses.

Ces propriétés justifient leur application en thérapeutique et l'invention a particulièrement pour objet à titre de médicaments, les produits de formule (I),
lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi plus particulièrement pour objet à titre de médicaments, les produits préférés tels que définis ci-dessus.

L'invention a tout particulièrement pour objet, à titre de médicaments, les produits décrits ci-après dans les exemples et notamment les produits de formule (I) telle que définie ci-dessus, dont les noms suivent :

- N-[3-(3-bromophényl)-2-(mercaptométhyl)-1-oxopropyl]-L-Tryptophane,
- N-(3-((1,2'-biphényl)-3-yl)-2-(mercaptométhyl)-1-oxopropyl]-L-Tryptophane,
- (R,S) N-(3-(4-bromophényl)-2-(mercaptométhyl)-1-oxopropyl] L-Tryptophane,
- (R,S) N-(3-((1,1'-biphényl)-4-yl)-2-(mercaptométhyl)-1-oxopropyl]-L-Tryptophane,
- (S) N-[2-(mercaptométhyl)-2-oxo-3-phénylpropyl]-4-(2-thiényl)-L-Phénylalanine,
- (S) N-[2-(mercaptométhyl)-1-oxo-3-phénylpropyl]-4-(1-naphtyl)-L-Phénylalanine,
- (S) N-(2-(mercaptométhyl) 1-oxo-3-phénylpropyl] L-Tryptophane,
- (S) 4-phényl-N-((2-mercaptométhyl)-1-oxo-3-phénylpropyl]-L-Phénylalanine,
- (S,S) N-(2-((1,1'-biphényl)-4-yl)-1-(1H-tétrazol-5-yl)-éthyl)-alpha-(mercaptométhyl)-benzènepropanamide,
- (R) et (S) N-[3-(3-bromophényl)-2-(mercaptométhyl)-1-oxopropyl]-4-(2-thiényl)-L-Phénylalanine (isomères A et B),
- N-[3-(3-bromophényl)-2-(mercaptométhyl)-1-oxopropyl]-7-(phénylméthoxy)-Tryptophane,

ainsi que leurs sels d'addition avec les acides minéraux ou organiques ou avec les bases minérales et organiques pharmaceutiquement acceptables desdits produits de formule (I).

Les médicaments, objet de l'invention, trouvent ainsi leur emploi dans le traitement, par utilisation d'un agent inhibiteur de l'enzyme de conversion de l'endothéline, d'affections telles que, par exemple, les spasmes vasculaires, le vasospasme consécutif à une hémorragie cérébrale, les spasmes coronariens, les spasmes vasculaires périphériques ainsi que les insuffisances rénales. Ces médicaments peuvent également être utilisés dans le traitement de l'infarctus du myocarde, de l'insuffisance cardiaque congestive, dans la prévention des resténoses post-angioplastie, des fibroses cardiaques et vasculaires, dans le traitement de l'athérosclérose, de certaines formes d'hypertension comme notamment l'hypertension pulmonaire, ainsi que dans le traitement de l'asthme.

Les médicaments, objet de l'invention, peuvent également trouver une application dans le traitement de l'ostéoporose, de l'hypertrophie prostatique et en tant que protecteurs neuronaux.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 300 mg par jour chez l'adulte, par voie orale ou de 1 à 100 mg par jour par voie intraveineuse.

Certains produits de départ de formules (II), (III), (XIIa), (XIIb), (VIII) et (X) sont connus ; ils peuvent être commerciaux ou être préparés selon les méthodes usuelles connues de l'homme du métier.

Certains produits de formule (III) peuvent être préparés par exemple comme indiqué dans le brevet européen EP 0465368.

On peut encore notamment préparer certains produits de formule (VIII) à partir d'autres produits de formule (VIII) par exemple en les soumettant à une ou plusieurs des réactions décrites ci-dessus, réalisées dans les conditions également décrites ci-dessus.

Des préparations de composés de formule (VIII) sont indiquées dans les exemples décrits ci-après.

Certains produits de départ de formule (VIII) peuvent être préparés également à partir de l'acide aminé tyrosine par modification selon les méthodes usuelles connues de l'homme du métier et notamment selon la méthode décrite dans la référence : Wen Ching Shieh et Coll., J. Org. Chem. 1992, 57, 379.

Ainsi on peut protéger la tyrosine sur ses fonctions amine et acide par exemple, respectivement, par estérification par un radical alkyle et sous forme de carbamate, puis former le triflate correspondant et le coupler en présence de Pd avec un dérivé boronique ou un autre organometallique. On prépare ainsi notamment à partir du radical

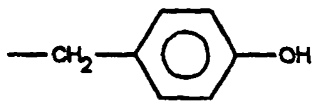

de la tyrosine, les produits de formule (VII) dans laquelle $R'_1$ représente notamment le radical phényle, substitué par un radical naphtyle, thiényle ou phényle, eux-mêmes éventuellement substitués comme indiqué ci-dessus dans la définition de $R_1$.

On peut noter que le composé de formule (VII) peut être sous forme racémique ou optiquement pure et que, en conséquence, les produits de formule (IX) obtenus peuvent également être sous forme de mélange de diastéréoisomères ou optiquement pure.

Des exemples de produits de départ de formules (XIIa) et (XIIb) sont donnés dans les préparations des exemples décrites ci-après.

La présente invention a enfin pour objet à titre de produits industriels nouveaux, les composés de formules (IX) et (XIV) telles que définies ci-dessus.

L'invention a particulièrement pour objet l'utilisation des produits de formule (I) telle que définie ci-dessus, pour la préparation d'un agent inhibiteur de l'enzyme de conversion de l'endothéline.

L'invention a ainsi particulièrement pour objet l'utilisation des produits de formule (I) telle que définie ci-dessus, pour la préparation de compositions pharmaceutiques destinées au traitement, par inhibition de l'enzyme de conversion de l'endothéline, d'affections telles que notamment l'hypertension induite par l'endothéline, les spasmes vasculaires, les suites d'une hémorragie cérébrale, les insuffisances rénales, l'infarctus du myocarde, l'insuffisance cardiaque ainsi que la prévention des resténoses post-angioplastie et des fibroses cardiaques et vasculaires.

Les exemples suivants illustrent l'invention sans toutefois la limiter.

**EXEMPLE 1 : N-[3-(3-bromophényl)-2-(mercaptométhyl)-1-oxopropyl]-L-Tryptophane**

STADE 1 : 3-bromo-alpha-[(diméthylamino)méthyl]-benzènepropanoate de méthyle

a) Préparation de LDA :

On introduit à -78°C dans 20 ml de tétrahydrofuranne anhydre, 4,7 ml de diisopropylamine et 23,75 ml de butyle lithium dans l'hexane à 15 %. La solution est agitée pendant 30 minutes à -78°C.

b) Réaction d'alkylation :

On ajoute ensuite 80 ml de tétrahydrofuranne anhydre, 10 ml de diméthylamino propanoate de méthyle, 4,35 ml de DMPU et 9 g de bromure de 3-bromobenzyle dissous dans 10 ml de tétrahydrofuranne anhydre. La solution est maintenue à -78°C pendant 3 heures puis ramenée à température ambiante. Après avoir ajouté 15 ml d'une solution saturée de chlorure d'ammonium, la solution est acidifiée à pH 1 avec de l'acide chlorhydrique molaire. Après extraction à l'éther, la phase aqueuse est amenée à pH 8 par l'ajout d'hydrogénocarbonate de sodium. On extrait ensuite à l'acétate d'éthyle, la phase organique est lavée à l'eau salée puis séchée. Après filtration, le solvant est évaporé puis le produit séché. On obtient ainsi 7,56 g de produit attendu (huile jaune), utilisé sans purification pour le stade suivant.

**Analyses physiques :**

RMN ($CDCl_3$/TMS, δ ppm)
2,1 (6H, s, $N(CH_3)_2$) ; 2,2 à 2,8 (5H, m, CH et $CH_2$) ; 3,5 (3H, s, ester méthylique) ; 6,9 à 7,4 (4H, m, aromatiques).

STADE 2 : iodure de 3-bromo-béta-(méthoxycarbonyl)-N,N,N-triméthyl-benzènepropanaminium

On introduit 3 g du produit obtenu au stade 1 ci-dessus, 30 ml d'isopropanol et ajoute 1,86 ml de iodure de

méthyle. La solution est agitée pendant une nuit. Il apparaît un précipité blanc qui est filtré, lavé avec un peu d'isopropanol, et séché. On obtient ainsi 3,81 g de produit attendu (poudre blanche).

**Analyses physiques :**

RMN ($CDCl_3$/TMS, δ ppm)
3,45 (9H, s, $N(CH_3)_3$) ; 3,7 (3H, s, ester méthylique) ; 3 (1H, d, $CH_2$) ; 3,9 (1H, dd, CH) ; 4,3 (2H, d, $CH_2$) ; 7,2 à 7,5 (4H, m, aromatiques).

STADE 3 : 3-bromo-alpha-méthylène-benzènepropanoate de méthyle

On mélange 3,81 g du produit obtenu au stade 2 ci-dessus avec 10 ml d'eau et 8,6 ml de soude 2N. La solution est portée au reflux à 100°C, pendant 2 heures. Après refroidissement, la solution est acidifiée avec de l'acide chlorhydrique molaire jusqu'à pH 1. Après filtration, rinçage à l'eau et séchage, on obtient 1,725 g de produit attendu.

**Analyses physiques :**

RMN ($CDCl_3$/TMS, δ ppm)
3,65 (2H, s, $CH_2$(4)) ; 5,65 (1H, s, H vinylique) ; 6,45 (1H, H vinylique) ; 7,2 à 7,4 (4H, m, aromatiques).

STADE 4 : acide alpha-[(acétylthio)méthyl]-3-bromo-benzène-propanoïque

On mélange 1,703 g du produit obtenu au stade 3 ci-dessus avec 1,75 ml d'acide thioacétique. Après 2 h d'agitation à température ambiante, la solution est chauffée pendant 1 h à 50°C puis l'acide thioacétique est évaporé sous vide. On obtient ainsi 2,217 g de produit attendu, utilisé sans purification pour l'étape suivante.

**Analyses physiques :**

IR dans $CHCl_3$ ($cm^{-1}$)

| | |
|---|---|
| OH acide | 3500 et absorption générale |
| C=O (acide et AcS) | 1747 - 1712 - 1696 |
| Aromatiques | 1598 - 1570. |

STADE 5 : ester éthylique du N-[2-[(acétylthio) méthyl]-3-(3-bromophényl)-1-oxopropyl]-L-Tryptophane

On introduit 1,88 g de L-Tryptophane sous forme de chlorhydrate dans 25 ml de chlorure de méthylène anhydre et ajoute 2,14 ml de triéthylamine, 3,1 g de BOP puis 2,217 g du produit obtenu au stade 4 ci-dessus, dissous dans 25 ml de chlorure de méthylène anhydre. La solution est agitée pendant 2 h à température ambiante, puis le solvant est évaporé, le produit obtenu sous forme d'une huile jaune, est ensuite purifié par chromatographie sur silice, avec comme éluant chloroforme-acétate d'éthyle : 85-15. Après évaporation du solvant et séchage, on obtient une gomme jaune. On obtient ainsi 2,69 g de produit attendu (mélange de deux diastéréoisomères).

**Analyses physiques :**

RMN ($CDCl_3$/TMS, δ ppm)
1,20 (3H, m, $CH_3$ ester éthylique) ; 2,35 et 2,38 (3H, deux singulets, AcS) ; 2,55 (1H, m, CH ou $CH_2$)) ; 2,80 à 3,30 (6H, m, $CH_2$ et CH) ; 4,10 (2H, m, $CH_2$ de l'ester éthylique) ; 4,90 (1H, m, CH) ; 5,95 et 6,00 (1H, deux doublets, NH) ; 6,45 et 7,00 (1H, deux doublets, H indolique) ; 7,10 à 7,65 (8H, m, aromatiques et indoliques) ; 8,10 (1H, d, NH indolique).

STADE 6 : N-[3-(3-bromophényl)-2-(mercaptométhyl)-1-oxopropyl]-L-Tryptophane

On ajoute à 200 mg du produit obtenu au stade 5 ci-dessus, dissous dans 2 ml de tétrahydrofuranne + 1 ml d'eau, 38 mg de lithine monohydrate. La solution est agitée pendant 1 h à température ambiante. 4 ml d'eau sont alors ajoutés puis la solution est acidifiée avec HCl 1N jusqu'à pH 1. Après avoir ajouté du chlorure de sodium, on extrait avec un mélange de chloroforme-méthanol : 80-20. La phase organique est ensuite séchée, puis le solvant évaporé.

Le produit brut, obtenu sous forme d'une mousse brun clair, est ensuite purifié par chromatographie sur silice, avec comme éluant chloroforme-méthanol : 90-10. On obtient ainsi 63 mg de produit attendu.

**Analyses physiques :**

RMN (DMSO/TMS, δ ppm)
2,19 à 3, 30 (9H, m, CH et $CH_2$) ; 4,38 (1H, m, CH) ; 6,74 à 7,60 (11H, aromatiques) ; 7,85 et 10,7 (deux doublets, H mobiles).

**EXEMPLE 2 : N-(3-(1,1'-biphényl)-3-yl)-2-(mercaptométhyl)-1-oxopropyl)-L-Tryptophane**

STADE 1 : 3-(1,1'-biphényl)-3-yl)-alpha-[(diméthylamino) méthyl]-propanoate de méthyle

On introduit 600 mg du produit obtenu au stade 1 de l'exemple 1, 350 mg de $Pd(P\phi_3)_2Cl_2$ dans 6 ml de toluène puis ajoute 636 mg de bicarbonate de sodium anhydre et 365 mg d'acide phényle boronique dissous dans 1,2 ml d'éthanol. La solution est agitée et portée au reflux à 100°C pendant 2 h. De l'eau salée est ajoutée à la solution et le produit est ensuite extrait à l'acétate d'éthyle. La phase organique est alors lavée à l'eau salée, séchée puis évaporée. Le produit obtenu sous forme d'une huile, est ensuite purifié par chromatographie sur silice, avec comme éluant chlorure de méthylène-méthanol : 93-7. Après évaporation du solvant et séchage, on obtient 462 mg de produit attendu (huile jaune).

STADE 2 : iodure de 3-(1,1'-biphényl)-3-yl)-béta-(méthoxycarbonyl)-N,N,N-triméthyl-propanaminium

On procède comme au stade 2 de l'exemple 1 à partir de 440 mg du produit obtenu au stade 1 ci-dessus, 6 ml d'isopropanol et 0,27 ml d'iodure de méthyle. On obtient ainsi 264 mg de produit attendu.

STADE 3 : 3-(1,1'-biphényl)-3-yl)-alpha-méthylène-propanoate de méthyle

On procède comme au stade 3 de l'exemple 1, à partir de 249 mg du produit obtenu au stade 2 ci-dessus, 3 ml d'eau et 0,55 ml de soude 2N. On obtient ainsi 111 mg de produit attendu (huile jaune).

STADE 4 : acide alpha-[(acétylthio)méthyl)-3-(1,1'-biphényl)-3-yl)propanoïque

On procède comme au stade 4 de l'exemple 1 à partir de 94 mg du produit obtenu au stade 3 ci-dessus et 0,07 ml d'acide thioacétique. On obtient ainsi 130 mg de produit attendu.

**Analyses physiques :**

RMN ($CDCl_3$/TMS, δ ppm)
2,4 (3H, s, AcS) ; 2,9 à 3,3 (5H, m, CH et $CH_2$) ; 7,2 à 7,7 (9H, m, aromatiques).

STADE 5 : ester éthylique du N-[2-[(acétylthio) méthyl]-3-(1,1'-biphényl)-3-yl)-1-oxopropyl]-L-Tryptophane

On procède comme au stade 5 de l'exemple 1, à partir de 91 mg de L-Tryptophane ester éthylique sous forme de chlorhydrate, 2 ml de chlorure de méthylène anhydre, 0,1 ml de triéthylamine, 150 mg de BOP et 107 mg du produit obtenu au stade 4 ci-dessus, dissous dans 25 ml de chlorure de méthylène-anhydre. On obtient ainsi 112 mg de produit attendu (gomme jaune).

STADE 6 : N-(3-(1,1'-biphényl)-3-yl)-2-(mercaptométhyl)-1-oxopropyl)-L-Tryptophane

On procède comme au stade 6 de l'exemple 1, à partir de 97 mg du produit obtenu au stade 5 ci-dessus, dissous dans 2 ml de tétrahydrofuranne, 1 ml d'eau et 23 mg de lithine monohydrate. On obtient ainsi 49 mg de produit attendu (mousse blanche).

**Analyses physiques :**

RMN (DMSO/TMS, δ ppm)
2,00 (1H, m large, SH mobile) ; 2,40 à 3,30 (7H, m, $CH_2$ et CH) ; 4,35 (1H, m, CH) ; 6,8 à 7,7 (14 H, m, aromatiques

et indoliques) ; 7,87-10,64 et 10,73 (3H, m larges, H mobiles).

**EXEMPLE 3 : N-[3-[6-chloro-1,3-benzodioxol-5-yl]-2-(mercaptométhyl)-1-oxopropyl] L-Tryptophane**

STADE 1 : (5-iodo-6-chloro)pipéronyle chloride

On introduit dans 180 ml d'acétone anhydre, 3,7 g de 6-chloropipéronyle chloride et 5,41 g d'iodure de sodium. La solution est portée au reflux pendant 2 h puis filtrée. Le solvant est évaporé, on élimine le chlorure de sodium en redissolvant dans l'acétate d'éthyle. Après évaporation, le produit est séché. On obtient ainsi 7,81 g de produit attendu.

**Analyses physiques :**

RMN ($CDCl_3$/TMS, δ ppm)
4,6 (2H, s, $CH_2I$) ; 6,05 (2H, s, O-$CH_2$-O) ; 6, 9 (1H, s, aromatique) ; 6,95 (1H, s, aromatique).

STADE 2 : 3-[6-chloro-1,3-benzodioxol-5-yl]-alpha-[(diméthylamino) méthyl]-propanoate de méthyle

On procède comme au stade 1 de l'exemple 1 à partir de 2,35 ml de diisopropylamine préalablement distillé, 11,8 ml de butyle lithium dans l'hexane à 15 %, 44 ml de tétrahydrofuranne, 2,2 ml de diméthylamino propanoate de méthyle, 2,2 ml de DMPU et 5,4 g du produit obtenu au stade 1 ci-dessus, dissous dans 10 ml de tétrahydrofuranne anhydre.

Le produit est purifié par chromatographie sur silice avec comme éluant chloroforme-méthanol : 95-5. On obtient ainsi 436 mg de produit attendu (huile jaune).

STADE 3 : iodure de 3-[6-chloro-1,3-benzodioxol-5-yl]-béta-(méthoxycarbonyl)-N,N,N-triméthyl-propanaminium

On procède comme au stade 2 de l'exemple 1, à partir de 436 mg du produit obtenu au stade 2 ci-dessus, 6 ml d'isopropanol et 0,27 ml d'iodure de méthyle. On obtient ainsi 501 mg de produit attendu.

STADE 4 : 3-[6-chloro-1,3-benzodioxol-5-yl]-alpha-méthylène-propanoate de méthyle

On procède comme au stade 3 de l'exemple 1 à partir de 484 mg du produit obtenu au stade 3 ci-dessus, 5 ml d'eau et 2 ml de soude 2N. On obtient ainsi 244 mg de produit attendu (poudre blanche).

STADE 5 : acide alpha-[(acétylthio)méthyl]-3-[6-chloro-1,3-benzodioxol-5-yl]-propanoïque

On procède comme au stade 4 de l'exemple 1 à partir de 223 mg de produit obtenu au stade 4 ci-dessus et 0,16 ml d'acide thioacétique. On obtient ainsi 172 mg de produit attendu.

**Analyses physiques :**

RMN ($CDCl_3$/TMS, δ ppm)
3,6 (2H, s, $CH_2$) ; 5,45 (1H, d, H vinylique) ; 5,95 (2H, s, O-$CH_2$-O) ; 6,35 (1H, d, H vinylique) ; 6,70 (1H, s, aromatique) ; 6,80 (1H, s, aromatique).

STADE 6 : ester éthylique du N-[2-[(acétylthio)méthyl]-3-(3-(6-chloro-1,3-benzodioxol-5-yl)-1-oxopropyl]-L-Tryptophane

On procède comme au stade 5 de l'exemple 1, à partir de 121 mg de L-Tryptophane sous forme de chlorhydrate, 2 ml de chlorure de méthylène anhydre, 0,13 ml de triéthylamine, 199 mg de BOP et 143 mg du produit obtenu au stade 5 ci-dessus, dissous dans 3 ml de chlorure de méthylène anhydre. On purifie par chromatographie sur silice avec comme éluant chloroforme-acétate d'éthyle : 90-10. On obtient ainsi 133 mg de produit attendu (gomme jaune).

STADE 7 : N-[3-[6-chloro-1,3-benzodioxol-5-yl]-2-(mercaptométhyl)-1-oxopropyl] L-Tryptophane

On procède comme au stade 6 de l'exemple 1 à partir de 123 mg du produit obtenu au stade 6 ci-dessus, dissous dans 2 ml de tétrahydrofuranne, 1 ml d'eau et 29 mg de lithine monohydrate. On purifie par chromatographie sur silice avec comme éluant chlorure de méthylène-méthanol-acide acétique : 89,5-10-0,5, le solvant est évaporé et

l'acide acétique éliminé. On obtient ainsi 53 mg de produit attendu (mousse blanche).

**Analyses physiques :**

RMN (DMSO/TMS, δ ppm)
1,87 et 2,18 (1H, SH mobile) ; 2,30 à 3,22 (7H, m, CH et $CH_2$) ; 4,46 et 4,52 (1H, m, CH) ; 5, 92 et 6,02 (2H, m O-$CH_2$-O) ; 6,83 (1H, m, aromatique) ; 6,92 à 7,33 (6H, m, aromatiques) ; 7,49 et 7,54 (1H, dl, aromatique) ; 8,26 (1H, m, CO-NH) ; 10,78 et 10,82 (1H, sl, NH indole) ; 12,60 (1H, m large, OH mobile).

**EXEMPLE 4 : (R,S)N-(3-(4-bromophényl)-2-(mercaptométhyl)-1-oxopropyl)-L-Tryptophane**

STADE 1 : 4-bromo-alpha-[(diméthylamino)méthyl]-benzènepropanoate de méthyle

On procède comme au stade 1 de l'exemple 1, en utilisant le 4-bromobenzyle bromide au lieu du 3-bromobenzyle bromide. Après purification sur silice avec pour éluant méthanol-chlorure de méthylène : 7-93, on obient 2,612 g de produit attendu (huile claire).

STADE 2 : iodure de 4-bromo-béta-(méthoxycarbonyl)-N,N,N-triméthyl-benzènepropanaminium

On procède comme au stade 2 de l'exemple 1 à partir de 1,093 g du produit obtenu au stade 1 ci-dessus, et obtient ainsi 1,267 g de produit attendu (solide blanc) utilisé tel quel au stade suivant.

STADE 3 : 4-bromo-alpha-méthylène-benzènepropanoate de méthyle

On procède comme au stade 3 de l'exemple 1 à partir de 1,267 g de produit obtenu au stade 2 ci-dessus et obtient ainsi 0,625 g de produit attendu (solide blanc).

STADE 4 : acide alpha-[(acétylthio)méthyl]-4-bromo-benzène-propanoïque

On procède comme au stade 4 de l'exemple 1 à partir de 0,62 g de produit obtenu au stade 3 ci-dessus et obtient ainsi 0,62 g de produit attendu (huile marron).

**Analyses physiques :**

IR dans $CHCl_3$ ($cm^{-1}$)

| | |
|---|---|
| >=O | 1746 - 1711 - 1695 |
| Aromatiques | 1580 - 1490. |

RMN ($CDCl_3$, δ ppm)
2,34 (3H, -$COCH_3$) ; 2,80 à 3,14 (5H, -$CH_2$-CH-$CH_2$-) ; 7,7 et 7,43 (m, aromatique)

STADE 5 : ester éthylique du N-[2-[(acétylthio)méthyl]-3-(4-bromophényl)-1-oxopropyl]-L-Tryptophane

On procède comme au stade 5 de l'exemple 1 à partir de 270 mg de L-Tryptophan-éthylester-hydrochloride, 9 ml de chlorure de méthylène, 0,3 ml de triéthylamine, 412 mg de BOP et 318 mg du produit obtenu au stade 4 ci-dessus, dissous dans 9 ml de chlorure de méthylène. On laisse 2 h sous agitation à température ambiante, évapore puis purifie sur silice avec pour éluant chloroforme-acétate d'éthyle : 85-15.
On obtient ainsi 295 mg de produit attendu (huile incolore).

STADE 6 : (R,S)N-(3-(4-bromophényl)-2-(mercaptométhyl)-1-oxopropyl)-L-Tryptophane

On procède comme au stade 6 de l'exemple 1 à partir de 150 mg du produit obtenu au stade 5 ci-dessus, 2 ml d'un mélange tétrahydrofuranne-eau : 2/1 et 28 mg d'hydroxyde de lithium monohydrate. On laisse quelques minutes à 0°C puis 1 h à température ambiante. On dilue à l'eau + acide chlorhydrique jusqu'à pH 1, extrait au chloroforme-méthanol: 80-20, sèche et évapore.
On purifie sur silice avec pour éluant chlorure de méthylène-méthanol-acide acétique : 89,5-10-0,5 et obtient

ainsi 100,5 mg de produit attendu (mousse blanche).

**Analyses physiques :**

RMN ($CDCl_3$, 1H) mélange de 2 diastéréoisomères
1,34 et 1,59 (t, 1H, SH) ; 2,30 à 2,50 (m, 2H) ; 2,63 à 2,91 (m, 3H) ; 3,19 et 3,31 (d, 2H) ; 4,85 (m, 1H, CHCO) ; 5,95 (d, 1H, NH) ; 6,59 et 7,0 (d, 1H, $H_2$ indole) ; 6, 87 à 7,60 (m, 8H, Ar) ; 8,12 et 8,16 (s1, 1H, NH indole).

**EXEMPLE 5 : (R,S)N-(3-((1,1'-biphényl)-4-yl)-2-(mercaptométhyl)-1-oxopropyl)-L-Tryptophane**

STADE 1 : 3-(1,1'-biphényl)-4-yl)-alpha-[(diméthylamino) méthyl]-propanoate de méthyle

On procède comme au stade 1 de l'exemple 2 à partir de 800 mg de parabromophényl, 112 mg de Pd $(PPh_3)_2Cl_2$, 7 ml de toluène, 4 ml d'une solution 2 mol/l de bicarbonate de sodium et 487 mg d'acide phénylboronique dans 1,6 ml d'éthanol, puis chauffe à reflux pendant 18 heures. On extrait à l'acétate d'éthyle, lave à l'eau, sèche et évapore les solvants. On purifie sur silice avec pour éluant acétate d'éthyle-méthanol : 93-7 et obtient ainsi 0,625 g de produit attendu (huile jaune).

**Analyses physiques :**

RMN (δ, $CDCl_3$, TMS)
2,26 (s, 6H, $-N(CH_3)_2$) ; 3,61 (s, 3H, $-CO_2CH_3$) ; 2,40 (dd, 2H) et 2,70 à 3,00 ($-CH_2-CH-CH_2-$) ; 7,22-7,49 (AA',BB', aromatique) ; 7,32 (m) 7,43 (m) 7,58 (d) (aromatique).

STADE 2 : iodure de 3-(1,1'-biphényl)-4-yl)-béta-(méthoxycarbonyl)-N,N,N-triméthyl-propanaminium

On procède comme au stade 2 de l'exemple 2 à partir de 624,4 mg du produit obtenu au stade 1 ci-dessus, et obtient ainsi 715 mg de produit attendu (solide blanc).

STADE 3 : 3-(1,1'-biphényl)-4-yl)-alpha-méthylène-propanoate de méthyle

On procède comme au stade 3 de l'exemple 2 à partir de 715 mg du produit obtenu au stade 2 ci-dessus, et obtient ainsi 393 mg de produit attendu (solide blanc).

STADE 4 : acide alpha-[(acétylthio) méthyl]-3-(1,1'-biphényl)-4-yl)-propanoïque

On procède comme au stade 4 de l'exemple 2 à partir de 350 mg du produit obtenu au stade 3 ci-dessus et obtient ainsi 405 mg de produit attendu.

**Analyses physiques :**

IR ($CHCl_3$) ($cm^{-1}$)

| | |
|---|---|
| >=0 | 1750 - 1700 |
| Aromatiques | 1600 - 1515 - 1487. |

STADE 5 : ester éthylique du N-[2-[(acétylthio) méthyl]-3-(3-(1,1'-biphényl)-4-yl)-1-oxopropyl]-L-Tryptophane

On procède comme au stade 5 de l'exemple 2 à partir de 200 mg du produit obtenu au stade 4 ci-dessus. Après purification sur silice avec pour éluant chloroforme-acétate d'éthyle : 85-15, on obtient 65 mg de produit attendu (huile marron).

STADE 6 : (R,S)N-(3-((1,1'-biphényl)-4-yl)-2-(mercaptométhyl)-1-oxopropyl)-L-Tryptophane

On procède comme au stade 6 de l'exemple 2, à partir de 65 mg du produit obtenu au stade 5 ci-dessus, purifie sur silice avec pour éluant chlorure de méthylène-méthanol-acide acétique : 89,5-10-0,5 et obtient ainsi 10 mg de produit attendu (huile marron).

**Analyses physiques :**

RMN ($CDCl_3$, 1H) mélange de 2 diastéréoisomères 50/50
1,36 et 1,59 (t, 1H, SH) ; 2,20 à 3,40 (m, 7H) ; 4,88 (m, CH) ; 5,94 (d, NH) ; 6,55 et 6,99 (d, 1H, $H_2$ indole) ; 6,80 à 7,90 (m, 13H).

**EXEMPLE 6 : (R,S) N-(2-(mercaptométhyl)1-oxo-3-(4-(phényl-méthoxy)phényl)propyl-L-Tryptophane**

STADE 1 : 3-(4-(phénylméthoxy) phényl)-alpha-[(diméthylamino) méthyl]-propanoate de méthyle

On procède comme au stade 1 de l'exemple 1, en utilisant au lieu du 3-bromobenzyle bromide, le 4-benzyloxybenzyle iodide, préparé à partir du 4-benzyloxybenzyle chloride selon les méthodes usuelles par action de l'hydrure de sodium dans l'acétone anhydre puis 2 h au reflux et purification sur silice. En procédant comme au stade 1 de l'exemple 1 à partir de 768 mg de diméthylamino propanoate de méthyle, on obtient une huile jaune que l'on purifie sur silice avec pour éluant chlorure de méthylène-méthanol : 92-8. On obtient ainsi 316 mg de produit attendu.

STADE 2 : iodure de 3-(4-(phénylméthoxy) phényl)-béta-(méthoxycarbonyl)-N,N,N-triméthyl-propanaminium

On procède comme au stade 2 de l'exemple 1 à partir de 315,8 mg du produit obtenu au stade 1 ci-dessus et obtient ainsi 246 mg de produit attendu utilisé tel quel au stade suivant.

STADE 3 : 3-(4-(phénylméthoxy)phényl)-alpha-méthylène-propanoate de méthyle

On procède comme au stade 3 de l'exemple 1 à partir de 246 mg du produit obtenu au stade 2 ci-dessus et obtient ainsi 136,11 mg de produit attendu (solide blanc).

STADE 4 : acide alpha-[(acétylthio)méthyl]-3-(4-(phényl-méthoxy) phényl)propanoïque

On procède comme au stade 4 de l'exemple 1, à partir de 136 mg du produit obtenu au stade 3 ci-dessus et obtient ainsi 149 mg de produit attendu (gomme marron).

**Analyses physiques :**

IR ($CHCl_3$) ($cm^{-1}$)

| | |
|---|---|
| >=O | 1693 |
| Aromatiques | 1611 - 1580 - 1512. |

STADE 5 : ester éthylique du N-[2-[(acétylthio)méthyl]-3-(4-(phénylméthoxy)phényl)-1-oxopropyl]-L-Tryptophane

On procède comme au stade 5 de l'exemple 1 à partir de 149 mg du produit obtenu au stade 5 ci-dessus, purifie sur silice avec pour éluant chloroforme-acétate d'éthyle : 85-15 et obtient ainsi 42 mg de produit attendu (huile).

STADE 6 : (R,S) N-(2-(mercaptométhyl) 1-oxo-3-(4-(phényl-méthoxy)phényl) propyl-L-Tryptophane

On procède comme au stade 6 de l'exemple 1 à partir de 42 mg du produit obtenu au stade 5 ci-dessus et obtient ainsi 16 mg de produit attendu (gomme jaune).

**Analyses physiques :**

RMN ($CDCl_3$, 1H, 300 MHz) mélange de 2 diastéréoisomères 50/50 1,31 et 1,55 (t, 1H, SH) ; 2,27 à 3,40 (m, 7H) ; 4,85 (m, 1H, CH) ; 5,90 (d, 1H, NH) ; 5,00 et 5,03 (s, 1H, O-$CH_2$) ; 6,51 et 7,0 (d, 1H) ; 6,8 à 7,6 (m, 17H) ; 7,99 et 8,06 (s1, 1H, NH).

**EXEMPLE 7 : (R) et (S) N-[3-(3-bromophényl)-2-(mercaptométhyl)-1-oxopropyl]-4-(2-thiényl)-L-Phénylalanine (isomères A et B)**

On procède comme à l'exemple 4, en utilisant au stade 5 de l'exemple 4, au lieu du L-Tryptophan-éthylester-hydrochloride, le composé correspondant dans lequel le radical indole est remplacé par un radical (1,1'-(thiényl) phényl-4-yl) ce composé étant préparé selon les méthodes usuelles (telles que par exemple dans la référence : J. Org. Chem. 1992, 57, 379-381), à partir de l'acide 2-thiényl boronique acide et de la N-boc-L-Tyrosine ester (comme indiqué au stade 1 de l'exemple 10 ci-dessus).

A l'issue du stade 5, on obtient ainsi un mélange des 2 stéréoisomères qui sont séparés par chromatographie sur silice avec pour éluant chloroforme-acétate d'éthyle : 90-10. On opère ensuite comme au stade 6 de l'exemple 4 à partir de chacun des diastéréoisomères ainsi séparés et obtient ainsi les deux produits attendus.

**Analyses :**

Isomère A :

$\alpha D$ = -24° à la concentration de 0,5 dans le diméthylformamide.
Spectre RMN (DMSO 250 MHz)
2,20 à 3,14 (7H) : les $CH_2$ et CH ; 4,27 (q, 1H) : CH ; 6,99 (2H, protons aromatiques) ; 7,10 à 7,50 (10H, les autres aromatiques) ; 7,72 (d) (1H, NH).

Isomère B :

$\alpha D$ = +17,3° à la concentration de 0,9 dans le diméthylformamide.
Spectre RMN (DMSO)
2,20 à 3,25 (7H, les $CH_2$ et CH) ; 4,27 (ml, 1H, CH) ; 7,00 à 7,60 (11H, les aromatiques).

**EXEMPLE 8 : N-[3-(3-bromophényl)-2-(mercaptométhyl)-1-oxopropyl]-7-(phénylméthoxy)-Tryptophane**

On procède comme à l'exemple 4, en utilisant au stade 5 de l'exemple 4, au lieu du L-Tryptophan-éthylester-hydrochloride, le composé correspondant dans lequel le radical indole est substitué sur le reste phényle de l'indole en ortho par un radical benzyloxy, ce composé étant commercial sous forme d'aminoacide.

On obtient ainsi le produit attendu.

**Analyses :**

Spectre RMN (DMSO 300 MHz)
5,23 (s, 2H, $OCH_2$) ; 2,2 à 3,5 (7H, $CH_2$ et CH) ; 4,40 (m, 1H, CH) ; 6,68 (m, 1H) - 6,85 (m, 1H) - 6,95 à 7, 55 (m, 11H, protons aromatiques) ; 8,05 (m, proton mobile 1H) ; 10,80 à 10,92 (m, 1H, proton mobile).

**EXEMPLE 9 : (S,S)M-(2-((1,1'-biphényl)-4-yl)-1-(1H-tétrazol-5-yl)éthyl)-alpha-(mercaptométhyl)-benzènepropanamide**

STADE 1 . [2-(R*,R*)] 2-[(acétylthio) méthyl] N-[2-[(1,1'-biphényl)-4-yl]-1-[1-(2-cyanoéthyl)-1H-tétrazol-5-yl] éthyl]-benzènepropanamide

On procède comme au stade 1 de l'exemple 16, en utilisant à l'étape b) au lieu du L-Tryptophan-méthylester, le composé (S) 5-[1-amino-2-[(1,1'-biphényl)-4-yl-] éthyl]-1H-tétrazole-1-propanenitrile, (préparé comme indiqué dans la référence J. of Bioorganic and Médicinal Chemistry Letters, 1995, p. 147).

On obtient ainsi le produit attendu.

**Analyses :**

$\alpha D$ = -32° dans le méthanol à la concentration de 0,5.
RMN : $CDCl_3$ 300 $MH_3$
6,94 à 7,14 (m, H, protons aromatiques sur phényl) ; -2,5 à 2, 75 (2H, $\underline{CH_2}$-phényle) ; 4,27 à 4, 45 (dt, 2H, -$\underline{CH_2}$-$CH_2$-CN) ; 3,37 (d, 2H, $CH_2$-biphényle) ; 2,45 à -2,70 (dt, 2H, $CH_2$-$\underline{CH_2}$-CN) ; 3,37 (d, 2H, $\underline{CH_2}$-biphényle) ; 6,17 (d, 1H, CON$\underline{H}$) ; 5,38 (q, 1H, $\underline{CH}$-$CH_2$-biphényle) ; -2,73 (m, 1H, -C$\underline{H}$-$CH_2$-phényle) ; 2,94 (d, 2H, CO-S-C$\underline{H_2}$-) ; 2,28

(s, 3H, $\underline{CH_3}$-CO-) ; 7,19 à 7,45 (système AA'-BB', Protons aromatiques biphényle).

STADE 2 : (S,S)N-(2-((1,1'-biphényl)-4-yl)-1-(1H-tétrazol-5-yl)éthyl)-alpha-(mercaptométhyl)-benzènepropanamide

On introduit 300 mg du produit obtenu au stade 1, 8 ml de méthanol, 4 ml de tétrahydrofuranne, 0,5 ml de 1,2-éthane dithiol, puis 5,6 ml de soude 1N.

On agite à température ambiante pendant 20 heures.

On dilue à l'eau, extrait une fraction neutre par une solution de chloroforme à 10 % de méthanol et acidifie la phase aqueuse puis extrait par du chloroforme à 10 % méthanol.
Après purification par chromatographie sur silice avec pour éluant chloroforme-méthanol : 90-10, on obtient 87 mg de produit attendu (produit amorphe incolore).
$\alpha D = \pm 0°$ à la concentration de 0,5 dans le méthanol.

**Analyses :**

RMN (DMSO 300 MHz)
5,45 (m, CO-NH-C$\underline{H}$-$CH_2$) - 8,59 (d, mobile, CO-N$\underline{H}$-CH-$CH_2$) ; 2,29 (m, 1H) - 2,60 (m, 3H) - 2,79 (m, 1H) - 3,16 (dd, 1H) - 3,30 (dd, 1H, les $CH_2$ et $CH_2$-CH-$CH_2$) ; 7,00 à 7,60 (14 protons aromatiques).

**EXEMPLE 10 : (S)N-[2-(mercaptaméthyl)-1-oxo-3-phénylpropyl] 4-(2-thiényl) L-phénylalanine**

STADE 1 : ester méthylique de la N-[(1,1-diméthyléthoxy) carbonyl]-4-(2-thiényl)-L-Phénylalanine

On introduit 417,5 mg d'ester méthylique de la N-[(1,1-diméthyléthoxy) carbonyl]-O-[(trifluorométhyl) sulfonyl] -L-Tyrosine (obtenu comme indiqué dans J. Org. Chem. 1992, 97, 379), 10 ml de toluène, 248 mg d'acide thiophène-2-boronique et 202 mg de bicarbonate de potassium, et agite la solution pendant 15 minutes à température ambiante. On ajoute à 90°C, 338,6 mg de tétrakis (thiphénylphosphine) Pd0 et chauffe la solution à 90°C pendant 3 heures. On ajoute 20 ml d'eau, extrait avec l'acétate d'éthyle, filtre la phase organique, effectue 2 lavages avec une solution de chlorure de sodium saturée, sèche les phases organiques. Après purification sur silice avec pour éluant (hexane-acétate d'éthyle : 6-4). On obtient 211 mg de produit attendu.

**Analyses physiques :**

IR ($CHCl_3$) ($cm^{-1}$).

| | |
|---|---|
| C-NH | 3438 |
| C=O | 1742 - 1710 |
| Système conjugué + Aromatique + Amide II | 1610 - 1567 - 1535 - 1501 |

STADE 2 : chlorhydrate de l'ester méthylique de la 4-(2-thiényl)-L-Phénylalanine

On mélange 277 mg du produit obtenu au stade 1 ci-dessus, 1,2 ml d'acétate d'éthyle et 2,77 ml d'une solution saturée d'acide chlorhydrique dans l'acétate d'éthyle, et agite à température ambiante pendant ≃ 2 h 15.
On concentre la solution, place à ≃ 4°C et sèche. On obtient ainsi 104,2 mg de produit attendu (cristaux blancs).

**Analyses physiques :**

RMN (**$CDCl_3$**, 1H, $\delta$ ppm)
7,14 (dd, 1H, Ar) ; 7,53 (m, 2H, Ar) ; 7,28 et 7,05 (2H, Ar) ; 4,32 (t, 1H, -CO-CH-N) ; 3,14 (dd, 2H) ; 8,53 (s, 3H, $NH_3$).

STADE 3 : Ester éthylique de la (S)N-[2-(acétylthio) méthyl]-1-oxo-3-phénylpropyl]-4-(2-thiényl)-L-Phénylalanine

On mélange 100 mg d'acide (S) alpha-[(acétylthio) méthyl]-benzènepropanoïque (dont la préparation et la

résolution sont décrites dans Journal of Pharmacology and Experimental Therapeutics 1987, 666, Vol. 243), avec 148 mg de BOP puis 80 mg du produit obtenu au stade 2 ci-dessus dissous dans 2 ml de chlorure de méthylène. On agite deux heures à température ambiante puis évapore. Après purification sur silice avec pour éluant chloroforme-acétate d'éthyle : 85-15, on obtient 122 mg de produit attendu (mousse blanche).

**Analyses physiques :**

RMN ($CDCl_3$)
2,35 (s, 3H, SAc) ; 2,5-3,2 (m, 7H, CH-$CH_2$) ; 3,7 (s, 3H, COOMe) ; 4,8 (q, 1H, CH) ; 5,8 (d, 1H, NH) ; 7,1-7,4 (m, 12H, Ar).

STADE 4 : (S)N-[2-(mercaptométhyl)-1-oxo-3-phénylpropyl] 4-(2-thiényl) L-phénylalanine

On introduit le produit obtenu au stade 3 ci-dessus, dans 1,5 ml de tétrahydrofuranne et ajoute 0,75 ml d'eau puis, après dégazage de la solution, 33 mg d'hydroxyde de lithium sont introduits à température ambiante. On agite 1,2 heures à température ambiante puis ajoute 4 ml d'eau, acidifie avec de l'acide chlorhydrique jusqu'à pH $\simeq$ 2. Après extraction au chlorure de méthylène et purification sur silice avec pour éluant chlorure de méthylène-méthanol-acide acétique : 10-89,5-0,5, on obtient 65 mg de produit attendu.

**Analyses physiques :**

Tgomme : 69-71°C
RMN (1H, $CDCl_3$, 300 MHz, δ)
1,40 (t, 1H, SH) ; 2,53 (m, 2H) ; 2,7 à 3,0 (m, 3H) ; 3,05 (dd, 1H) ; 3,22 (dd, 1H) ; 4,85 (m, 1H, CH) ; 5,90 (m, 1H, NH) ; 6,80 à 7,50 (m, 12H, H aromatiques).

**EXEMPLE 11 : (S)N-[2-(mercaptométhyl)-1-oxo-3-phénylpropyl] 4-(1-naphtyl)-L-phénylalanine**

STADE 1 : ester méthylique de la N-[(1,1-diméthyléthoxy) carbonyl]-4-(1-naphtyl)-L-Phénylalanine

On procède comme au stade 1 de l'exemple 10 à partir d'ester méthylique de la N-[(1,1-diméthyléthoxy) carbonyl]-O-[(trifluorométhyl) sulfonyl]-L-tyrosine (préparé comme indiqué au stade 1 de l'exemple 10), 10 ml de toluène, 282 mg d'acide naphtalène boronique et 169,7 mg de bicarbonate de sodium, agite la solution pendant 15 minutes, puis ajoute 94,77 mg de 5-tétrakis (triphénylphosphine). On obtient ainsi 165,7 mg de produit attendu.

**Analyses physiques :**

IR $CHCl_3$ ($cm^{-1}$)

| | |
|---|---|
| NH | 3435 |
| >=O | 1742 - 1711 |
| Aromatique + Amide II | 1600 - 1590 - 1503 |

STADE 2 : Chlorhydrate de l'ester méthylique de la 4-(1-naphtyl)-L-Phénylalanine

On procède comme au stade 2 de l'exemple 10 à partir du produit obtenu au stade 1 ci-dessus et obtient le produit attendu.

STADE 3 : ester méthylique de la N-[2-[(acétylthio)méthyl] 1-oxo 3-phénylpropyl]-4-(1-naphtyl)-L-Phénylalanine

On procède comme au stade 3 de l'exemple 10 à partir du produit obtenu au stade 2 ci-dessus et obtient le produit attendu.

STADE 4 : (S)N-[2-(mercaptométhyl)-1-oxo-3-phénylpropyl] 4-(1-naphtyl)-L-phénylalanine

On procède comme au stade 4 de l'exemple 10 à partir du produit obtenu au stade 3, ci-dessus et obtient le produit attendu. F = 158°C.

**Analyses physiques :**

RMN (DMSO, 300 MHz, δ)
1,95 (t, 1H, SH) ; 2,27 (m, 1H) ; 2,50 à 3,0 (m, 5H) ; 3,27 (dd, 1H) ; 4,40 (m, dd après échange, 1H, CH-NHCO) ; 7,08 à 8,0 (m, 18H, H aromatiques + CONH).

**EXEMPLE 12 : (S)N-[2-(mercaptométhyl)-1-oxo-3-phénylpropyl] 4-(4-méthoxyphényl) L-phénylalanine**

STADE 1 : ester méthylique de la N-[(1,1-diméthyléthoxy) carbonyl]-4-(4-méthoxyphényl)-L-Phénylalanine

On procède comme au stade 1 de l'exemple 10 en utilisant au lieu de l'acide thiophène-2-boronique, l'acide 4-méthoxy benzèneboronique à partir de 351,6 mg d'ester méthylique de la N-[(1,1-diméthyléthoxy) carbonyl]-O-[(trifluorométhyl) sulfonyl]-L-tyrosine (préparé comme indiqué au stade 1 de l'exemple 10), 10 ml de toluène, 250 mg d'acide 4-méthoxy-benzène boronique, 170,3 mg de bicarbonate de potassium et 190,17 mg de 5-tétrakis (triphényl phosphine) Pd(O). On obtient ainsi 199 mg de produit attendu.

**Analyses physiques :**

IR $CHCl_3$ ($cm^{-1}$)

| | |
|---|---|
| NH | 3440 |
| C=O | 1742 - 1710 |
| Aromatiques + Amide II | 1610 - 1500 |

STADE 2 : chlorhydrate de l'ester méthylique de la 4-(4-méthoxyphényl)-L-Phénylalanine

On procède comme au stade 2 de l'exemple 10 à partir de 200 mg du produit obtenu au stade 1 ci-dessus, 1 ml d'acétate d'éthyle et 1,8 ml d'une solution saturée d'acide chlorhydrique dans l'acétate d'éthyle. On obtient ainsi 81 mg de produit attendu.

**Analyses physiques :**

RMN ($CDCl_3$ + quelques gouttes de MeOD/TMS, δ ppm)
3,15 (2H, d, $CH_2$) ; 3,67 (3H, s, O-Me) ; 3,7 (3H, s, ester méthylique) ; 4,1 (1H, t, CH) ; 6,82 (2H, d, aromatiques) ; 7,14 (2H, d, aromatiques) ; 7, 36 (2H, d, aromatiques) ; 7,4 (2H, d, aromatiques).

STADE 3 : ester méthylique de la (S)N-[2-[(acétylthio) méthyl]-1-oxo-3-phénylpropyl]-4-(4-méthoxyphényl)-L-Phénylalanine

On procède comme au stade 3 de l'exemple 10, à partir de 80 mg d'acide (S) alpha-[(acétylthio) méthyl]-benzènepropanoïque (préparé selon la référence indiquée au stade 3 de l'exemple 10), 2 ml de chlorure de méthylène, 0,07 ml de triéthylamine, 106 mg de BOP et 60 mg du produit obtenu au stade 2 ci-dessus, dissous dans 1 ml de chlorure de méthylène. On obtient ainsi 82 mg de produit attendu.

STADE 4 : (S)N-[2-(mercaptométhyl)-1-oxo-3-phénylpropyl]-4-(4-méthoxyphényl)-L-phénylalanine

On procède comme au stade 4 de l'exemple 10 à partir de 68 mg du produit obtenu au stade 3 ci-dessus, dissous dans 2 ml de tétrahydrofuranne, 1 ml d'eau et 17 mg de lithine monohydrate. On obtient ainsi 40 mg de produit attendu (solide blanc).

**Analyses physiques :**

RMN (DMSO/TMS, δ ppm)
1,81 (1H, m large, SH mobile) ; 2,29 (1H, m, CH) ; 2,50 à 3,20 (6H, m, $CH_2$) ; 3,78 (3H, s, OMe) ; 4,49 (1H, m, CH) ; 8,29 (1H, dl, CO-NH mobile) ; 7,00 (2H, dl, aromatiques) ; 7,10 à 7,30 (8H, m, aromatiques) ; 7,25 à 7,60 (3H, m, aromatiques) ; 12,80 (1H, m large, OH mobile).

**EXEMPLE 13 : (S)N-[2-(mercaptométhyl)-1-oxo-3-phénylpropyl] béta-phényl-phénylalanine**

STADE 1 : trifluorométhanesulfonate de l'ester méthylique de la béta-phényl-L-Phénylalanine

On procède comme au stade 2 de l'exemple 10 à partir de 500 mg d'ester méthylique de la N-[(1,1-diméthyléthoxy) carbonyl] béta-phényl-L-Phénylalanine (préparé à partir de la LBoc 3-3-diphénylalanine (commercial) par estérification selon des méthodes connues de la chimie peptidique: par exemple EDC, MeOH, $CH_2Cl_2$), 10 ml de chlorure de méthylène et 5 ml d'acide trifluoroacétique. On obtient ainsi 361 mg de produit attendu (solide jaune).

**Analyses physiques :**

RMN ($CDCl_3$ + quelques gouttes de MeOD/TMS, $\delta$ ppm)
3,33 (3H, s, ester méthylique) ; 4,13 (1H, d, CH) ; 4,8 (1H, d, CH) ; 6, 96 à 7,08 (10H, m, aromatiques).

STADE 2 : ester méthylique de la (S)N-[2-[(acétylthio) méthyl]-1-oxo-3-phénylpropyl]-béta-phényl-L-Phénylalanine

On procède comme au stade 3 de l'exemple 10 à partir de 221 mg d'acide (S) alpha-[(acétylthio) méthyl]-benzènepropanoïque (préparé selon la référence indiquée au stade 3 de l'exemple 10), 343 mg du produit obtenu au stade 1 ci-dessus, 8 ml de chlorure de méthylène anhydre, 0,28 ml de triéthylamine et 410 mg de BOP.
On obtient ainsi 372 mg de produit attendu (mousse blanche).

STADE 3 : (S)N-[2-(mercaptométhyl)-1-oxo-3-phénylpropyl] béta-phényl-phénylalanine

On procède comme au stade 4 de l'exemple 10 à partir de 200 mg du produit obtenu au stade 2 ci-dessus, dissous dans 2 ml de tétrahydrofuranne, 1 ml d'eau et 53 mg de lithine monohydrate. On obtient ainsi 165 mg de produit attendu (mousse blanche).
Pouvoir rotatoire : $[\alpha]_D$=+12° à la concentration de 0,5 dans le chloroforme.

**Analyses physiques :**

IR $CHCl_3$ ($cm^{-1}$)

| | |
|---|---|
| =C-NH | 3428 |
| >=O | 1753 - 1719 - 1678 |
| SH | 2568 |
| Aromatiques et amide II | 1603 - 1585 - 1510 - 1497 |

Absorption générale type acide.

**EXEMPLE 14 : (S)N-[2-(mercaptométhyl)-1-oxo-3-phénylpropyl] S-phényl-L-Cystéine**

STADE 1 : ester méthylique de la S-phényl-L-Cystéine

On procède de façon analogue à la méthode décrite dans Tet. Lett., 1995, 36, 4133, en partant de la NBOC L Cystéine méthylester, suivi d'une déprotection de l'azote selon une technique classique (par exemple TPA/$CH_2Cl_2$) et obtient ainsi le produit attendu.

STADE 2 : ester méthylique de la (S)N-[2-[(acétylthio) méthyl]-1-oxo-3-phénylpropyl]-S-phényl-L-Cystéine

On procède comme au stade 3 de l'exemple 10, à partir de 400 mg d'acide (S) alpha-[(acétylthio) méthyl]-benzènepropanoïque (préparé selon la référence indiquée au stade 3 de l'exemple 10), 5 ml de chlorure de méthylène, 0,36 ml de triéthylamine, 521 mg de BOP et 280 mg du produit obtenu au stade 1 ci-dessus dissous dans 5 ml de chlorure de méthylène.
On obtient ainsi 389 mg de produit attendu.

STADE 3 : (S)N-[2-(mercaptométhyl)-1-oxo-3-phénylpropyl] S-phényl-L-Cystéine

On introduit 128 mg de produit obtenu au stade 2 ci-dessus, 0,38 ml de soude molaire et 2 ml de méthanol. La solution est acidifiée à pH 1 par ajout d'acide chlorhydrique molaire, puis extraite avec un mélange binaire de chlorure de méthylène et d'acétate d'éthyle. Après séchage et évaporation, le produit est purifié par chromatographie sur silice avec pour éluant chlorure de méthylène-méthanol-acide acétique : 89,5-10-0,5. On obtient ainsi 73 mg de produit attendu (mousse blanche).

**Analyses physiques :**

RMN ($CDCl_3$/TMS, $\delta$ ppm)
1,56 (1H, t, SH mobile) ; 2,45 à 2,95 (5H, m, CH et $CH_2$) ; 3,30 (1H, dd, J = 14 et 6, H de $CH_2$) ; 3,46 (1H, dd, J = 14 et 4,5, H de $CH_2$) ; 4,72 (1H, dd après échange, CH) ; 6,14 (1H, d, NH de l'amide mobile) ; 7,10 à 7,41 (10H, m, aromatiques).
Pouvoir rotatoire : $[\alpha]_D$=+30,4° à la concentration 0,5 dans le chloroforme.

**EXEMPLE 15 : (S)N-[2-(mercaptométhyl)-1-oxo-3-phénylpropyl] 5-(phénylméthoxy)-D,L-Tryptophane**

STADE 1 : ester méthylique du 5-(phénylméthoxy)-D,L-Tryptophane

On introduit 300 mg de Tryptophane-5-O-benzyl racémique (commercial) dans un mélange de 15 ml de méthanol et de 3 ml de chlorure d'acétyle, laisse 24 h à température ambiante, puis saponifie jusqu'à ph ≃ 8 et extrait à l'acétate d'éthyle. Après chromatographie sur silice avec pour éluant chlorure de méthylène-méthanol : 90-10, on obtient 210 mg d'ester attendu.

STADE 2 : ester méthylique du (S)N-[2-(acétylthio)méthyl)-1-oxo-3-phénylpropyl)-5-(phénylméthoxy)-D,L-Tryptophane

On procède comme au stade 3 de l'exemple 10, à partir de 150 mg du produit obtenu au stade 2 ci-dessus. On obtient ainsi 280 mg de produit attendu.

STADE 3 : (S)N-[2-(mercaptométhyl)-1-oxo-3-phénylpropyl)-5-(phénylméthoxy)-D,L-Tryptophane

On procède comme au stade 4 de l'exemple 10 et obtient ainsi 150 mg de produit attendu.

**Analyses physiques :**

Produit sous forme de deux diastéréoisomères
Mousse blanche Tgomme : 67-69°C
IR $CHCl_3$ ($cm^{-1}$)

| | |
|---|---|
| =C-NH | 3480 - 3424 |
| C=O | 1754 - 1720 - 1673 |
| SH | 2567 |
| hétérocycles + aromatiques + amide II | 1625 - 1604 - 1585 - 1511 - 1498 - 1484 |

RMN ($CDCl_3$/300 MHz)

Mélange de deux diastéréoisomères
1,34 et 1,54 (t, 1H, SH) ; 2,46 (m, 2H) ; 2,7 à 3,25 (m, 5H) ; 4,81 (m, 1H) ; 5,09 (m, 2H) ; 5,94 (m, 1H) ; 6,41 (sl, 1H, $H_2$ indole) ; 6,90 à 7,47 (m, 15H) ; 7,90 et 8,01 (s1, 1H, NH indole).

**<u>EXEMPLE 16</u> : ester méthylique du (S)N-[2-(mercaptométhyl)-1-oxo-3-phénylpropyl]-L-Tryptophane**

<u>STADE 1</u> : Acide (S) alpha-[(acétylthio)méthyl)-benzènepropanoïque

a) Préparation du chlorure d'acide

On introduit 615 mg d'acide (obtenu comme indiqué dans la référence : Journal of Pharmacology and Expérimental Therapeutics 1987, 666, vol. 243), 0,4 ml de chlorure de thionyle, agite à la température ambiante pendant 2 heures puis chasse le chlorure de thionyle.

b) Réaction d'amidification

On dissout le résidu obtenu ci-dessus en a) dans 3 ml de chlorure de méthylène puis ajoute sous agitation, 300 mg de DMAP et lentement une solution de 650 mg de L-trytophane méthylester (libéré de son chlorhydrate) et 6 ml de chlorure de méthylène. On agite à température ambiante pendant 2 heures, dilue à l'eau, décante et poursuit l'extraction par du chlorure de méthylène.

c) Après purification du produit obtenu ci-dessus en b), par chromatographie sur silice avec pour éluant chloroforme-acétate d'éthyle : 98-2, on obtient 574 mg de produit attendu (produit amorphe incolore).

$[\alpha]_D$=-44°, à la concentration de 0,5 dans le méthanol.
RMN ($CDCl_3$)
2,75 à 3,10 (m, 4H, les 3 $CH_2$), 3,24 (d, 2H) ; 7,02 à 7,40 (m, 7H, protons aromatiques) ; 7,34 (dl, 1H), 7,53 (dl, 1H).

<u>STADE 2</u> : ester méthylique du (S)N-[2-(mercaptométhyl)-1-oxo-3-phénylpropyl]-L-Tryptophane

On introduit 150 mg du produit obtenu au stade 1 ci-dessus, 10 ml de méthanol, refroidit à 0°C et introduit lentement 3,6 ml de soude 0,1 N. Puis on agite à 0°C pendant deux heures. On chasse le méthanol sous vide à température ambiante et extrait par du chloroforme à 20 % de méthanol. Après purification par chromatographie sur silice avec pour éluant chloroforme-acétate d'éthyle 98-2, on obtient 78 mg de produit attendu (produit amorphe incolore).
$[\alpha]_D$=+14,4°, à la concentration de 0,5 dans le méthanol.

**Analyses physiques :**

Microanalyse :

| | Calculé | Trouvé |
|---|---|---|
| C % | 66,64 | 67,1 |
| H % | 6,10 | 6,3 |
| N % | 7,06 | 6,2 |
| S % | 8,09 | 7,6 |

IR $CHCl_3$ ($cm^{-1}$)

| | |
|---|---|
| =C-NH | 3479 - 3404 |
| C=O | 1740 |
| Hétérocycle + Aromatique | 1620 - 1604 - 1585 - 1553 - 1511 - 1401 |

RMN ($CDCl_3$/300 MHz)
2,49 (m, 2H, les (<u>$CH_2$</u>)-CH et $CH_2$-<u>CH</u>-$CH_2$) ; 2,70 à 2,95 (m, 3H) ; 3,29 (d, 2H) ; 7,05 à 7,38 (m, les protons aromatiques) ; 7,56 (d, 1H).

**<u>EXEMPLE 17</u> : (S)N-[2-(mercaptométhyl)-1-oxo-3-phenylpropyl] L-Tryptophane**

La phase aqueuse du produit de l'exemple 16 est acidifiée par addition d'acide chlorhydrique et on extrait le précipité gommeux obtenu par du chloroforme à 20 % de méthanol. Pour purification, on dissout les 59 mg ainsi obtenus dans 5 ml de soude 0,1N, acidifie par de l'acide chlorhydrique, filtre, lave et sèche sous pression réduite à température ambiante. On obtient ainsi 42 mg de produit attendu (produit amorphe, incolore). $[\alpha]_D$=+15,2°, à la concentration de 0,5 dans le méthanol.

**Analyses :**

IR $CHCl_3$ ($cm^{-1}$)

| | |
|---|---|
| =C-NH | 3477 - 3423 |
| + absorption générale | |
| C=O | 1722 - 1669 |
| Hétérocycle | 1604 - 1585 - 1514 - 1498 |
| + Aromatique + Amide II | |

**<u>EXEMPLE 18</u> : ester méthylique du (R)N-[2-(mercaptométhyl)-1-oxo-3-phénylpropyl]-L-Tryptophane**

<u>STADE 1</u> : ester méthylique du (R)N-[2-[(acétylthio) méthyl]-1-oxo-3-phénylpropyl]-L-Tryptophane

On procède comme au stade 1 de l'exemple 16 en utilisant, à la place du sel d'éphédrine lévogyre, le sel d'éphédrine dextrogyre (F=130°C, $[\alpha]_D$=+49,6°C, à la concentration 0,5 dans le méthanol. On obtient ainsi 525 mg de produit attendu (produit amorphe incolore). $[\alpha]_D$=+32,8°, à la concentration 0,5 dans le méthanol.

**Analyses physiques :**

RMN $CDCl_3$
2,29 (s, <u>CH</u>$_3$-CO) ; 2,80 à 3,00 (m, 3H) ; 3,20 (dd, 1H) ; 3,08 (d, 2H) ; 2, 54 (m) ; 3,60 (s, 1H) ; 4, 87 (m, 1H) ; 5, 89 (dl peu mobile) ; 6, 89 (d, sl après échange) ; 7, 92 (sl, peu mobile) ; 7,00 à 7,31 (m, 9H, Aromatiques).

<u>STADE 2</u> : ester méthylique du (R)N-[2-(mercaptométhyl)-1-oxo-3-phénylpropyl] L-Tryptophane

On procède comme au stade 2 de l'exemple 16, à partir de 150 mg de produit obtenu au stade 1 ci-dessus, 10 ml de méthanol et 3,6 ml de soude 0,1N. On obtient ainsi 60 mg de produit attendu (solide amorphe rosé). $[\alpha]_D$=-17,6° à la concentration de 0,5 dans le méthanol.

**Analyses physiques :**

Microanalyse :

| | Calculé | Trouvé |
|---|---|---|
| C % | 66,64 | 66,5 |
| H % | 6,10 | 6,1 |
| N % | 7,06 | 6,8 |
| S % | 8,09 | 8,0 |
| O % | 12,11 | |

IR $CHCl_3$ ($cm^{-1}$)

| | |
|---|---|
| =C-NH | 3479 - 3424 |
| C=0 | 1740 - 1672 |
| Hétérocycle + Aromatique + Amide | 1620 - 1604 - 1585 - 1553 - 1511 - 1497 |

Faible absorption - 2870 (région SH).

**EXEMPLE 19 : (R)N-[2-(mercaptométhyl)-1-oxo-3-phénylpropyl] L-Tryptophane**

On procède comme à l'exemple 17 à partir du produit obtenu au stade 1 de l'exemple 18, et obtient ainsi 54 mg de produit attendu (solide incolore). $[\alpha]_D$=-18,4°, à la concentration 0,5 dans le méthanol.

**Analyses physiques :**

Microanalyse :

| | Calculé | Trouvé |
|---|---|---|
| C % | 65,95 | 65,2 |
| H % | 5,80 | 5,7 |
| N % | 7,32 | 7,1 |
| S % | 8,38 | 8,1 |
| O % | 12,55 | |

IR $CHCl_3$ ($cm^{-1}$)

| | |
|---|---|
| =C-NH | 3478 - 3420 |
| C=0 | 1735 - 1722 - 1669 |
| Hétérocycle + Aromatique + Amide II | 1621 - 1604 - 1585 - 1514 - 1498 |

Faible absorption à 2580 (région SH).

**EXEMPLE 20 : (S)N-[2-(mercaptométhyl)-1-oxo-3-phénylpropyl]-L-phénylalanine**

STADE 1 : ester (1,1-diméthyléthylique) de la (S)N-[2-[(acétylthio) méthyl]-1-oxo-3-phénylpropyl]-L-Phéaylalanine

On procède comme aux étapes a, b et c du stade 1 de l'exemple 16 en utilisant à l'étape a) le même sel d'éphédrine lévogyre et à l'étape c), au lieu de la L-Tryptophane méthylester, l'ester terbutylique de la L-phénylalanine (libéré de son chlorhydrate). On obtient ainsi le produit attendu $[\alpha]_D$=-44°, à la concentration 0,5 dans le méthanol.

**Analyses physiques :**

RMN :
5,79 (dl) ; 4,59 (m) ; 2,34 (s) ; 2,55 (m) 1H ; 2,76 à 3,10 (m) 7H ; 1,33 (s) ; 7,10 à 7,25 (m, Protons aromatiques).

STADE 2 : (S)N-[2-(mercaptométhyl)-1-oxo-3-phénylpropyl]-L-phénylalanine

a) Déblocage de la fonction acide :

On introduit 370 mg du produit obtenu au stade 1 ci-dessus, et 4 ml de chlorure de méthylène, refroidit à 0°C et introduit 2 ml d'acide trifluoroacétique. On agite 1h à 0°C, puis à température ambiante pendant 2 heures.
Après avoir chassé solvants et réactifs, on purifie par chromatographie sur silice avec pour éluant chloroforme-méthanol : 90-10. On obtient ainsi 246 mg de produit attendu (produit jaune paille) amorphe. $[\alpha]_D$=-7,2°, à la concentration 0,5 dans le méthanol.

b) Clivage du thioacétate :

On introduit 100 mg de l'acide obtenu en a) ci-dessus et 3 ml de méthanol, refroidit à 0°C, introduit 3 ml de soude 0,1 N et agite à 0°C pendant 2 heures. On chasse le méthanol, dilue à l'eau, acidifie par addition d'acide chlorhydrique 2N, filtre, lave à l'eau et sèche. On obtient ainsi 68 mg de produit attendu (produit amorphe incolore). $[\alpha]_D$=+8°, à la concentration 0,5 dans le méthanol.

**Analyses physiques :**

IR $CFiCl_3$ ($cm^{-1}$)

| | |
|---|---|
| =C-NH | 3428 |
| C=O | 1722 (acide) - 1672 (amide) |
| Aromatiques + Amide II | 1604 - 1585 - 1513 - 1497 |

Faible absorption à 2570 (région SH).

**EXEMPLE 21 : (S) 4-phényl-N-((2-(mercaptométhyl)-1-oxo-3-phénylpropyl]-L-phénylalanine**

STADE 1 : ester méthylique de la 4-phényl-L-Phénylalanine

a) Préparation du triflate

On introduit 1,18 g de N-BOC-L-tyrosine méthyl ester (S), 6 ml de chlorure de méthylène et 1,6 ml de pyridine. On refroidit à 0-5°C la solution obtenue puis introduit 0,8 ml d'anhydride trifluorométhane sulfonique. On agite 1h à 0°C, reprend à l'eau et extrait par du chlorure de méthylène), lave à la soude 1N puis par de l'acide chlorhydrique 1N et enfin à l'eau salée. On filtre et sèche.
Après purification par chromatographie avec pour éluant hexane-acétate d'éthyle : 70-30, on récupère 161 g du produit attendu (huile jaune paille). $[\alpha]_D$=+34,8°, à la concentration de 1 dans le chloroforme.

**Analyses physiques :**

RMN $CDCl_3$
1,5 (s, O-terbut) ; 3,75 (s, $OCH_3$) ; ≃ 5,0 (NH) ; ≃ 4,65 (CH) ; 3 à 3,3 ($CH_2$).

b) Réaction de couplage :

On introduit 1,6 g du triflate préparé en a) ci-dessus, 35 ml de toluène, 920 mg d'acide benzène boronique à 98 % et 770 mg de bicarbonate de potassium. On agite 15 mn à température ambiante puis introduit 115 mg de tétrakis (triphénylphosphine) palladium (Pd°) et chauffe à 90°C sous agitation pendant 2 h 30.
On dilue à l'eau et extrait à l'acétate d'éthyle, sèche, filtre la phase organique et chasse les solvants.
Après purification par chromatographie sur silice avec pour éluant hexane-acétate d'éthyle : 70-30, on récupère 1,4 g de résine jaune. $[\alpha]_D$=+48°, à la concentration de 1 dans le chloroforme.

c) Réaction de déblocage de la fonction amine

On introduit 1,4 g du produit obtenu en b) ci-dessus, 6 ml d'acétate d'éthyle puis 14 ml d'une solution saturée d'acide chlorhydrique dans l'acétate d'éthyle et agite à température ambiante pendant 3 heures, puis concentre, glace, filtre et sèche. On obtient ainsi 960 mg de produit attendu (cristaux incolores) F = 230-232°C. $[\alpha]_D$=+11,6°, à la concentration de 1 dans le méthanol.

**Analyses physiques :**

IR Nujol ($cm^{-1}$)

| | |
|---|---|
| C=O<br>$NH_2$ déf. + aromatique | 1738<br>1595 - 1576 - 1518 - 1500 |

STADE 2 : ester méthylique de la (S)N-[2-[(acétylthio) méthyl]-1-oxo-3-phénylpropyl]-4-phényl-L-Phénylalanine

On procède comme au stade 1 de l'exemple 16 en utilisant à l'étape c), le produit obtenu au stade 1 ci-dessus au lieu du L-Tryptophane méthylester. On obtient ainsi le produit attendu (cristaux incolores). F = 125°C. $[\alpha]_D$=-60°, à la concentration 0,5 dans le méthanol.

**Analyses physiques :**

IR $CHCl_3$ ($cm^{-1}$)

| | |
|---|---|
| =C-NH | 3420 |
| C=O | 1742 - 1682 |
| aromatique + Amide II | } 1600 - 1580 - 1510 - 1487 |

STADE 3 : (S)4-phényl-N-((2-(mercaptométhyl)-1-oxo-3-phénylpropyl]-L-phénylalanine

On introduit 200 mg du produit obtenu au stade 2 ci-dessus, 3 ml d'un mélange tétrahydrofuranne/eau : 2/1 et 40 ml d'hydroxyde de lithium monohydrate et agite à température ambiante pendant deux heures. Puis on dilue à l'eau, acidifie par de l'acide chlorhydrique et extrait par du chloroforme à 20 % de méthanol.
Après purification par chromatographie sur silice avec pour éluant chloroforme-méthanol 90-10, on obtient 31 mg de produit attendu (solide incolore). $[\alpha]_D$=+22°, à la concentration 0,5 dans le méthanol.

**Analyses physiques :**

**RMN DMSO**
1,90 (m, 1H, SH) ; 2,20 à 3, 30 (m, 7H) ; 4, 36 (m, 1H) ; 7,08 à 7,60 (m, 14H) ; 7, 93 (m, 1H, NH) ; 7,02 à 7,60 (m) - 14 aromatiques.

**EXEMPLE 22 : (S)N-(2-mercaptométhyl)-1-oxo-3-phénylpropyl)-O-phénylméthyl-L-Tyrosine**

STADE 1 : ester méthylique de la N-[2-[(acétylthio) méthyl]-1-oxo-3-phénylpropyl]-O-(phénylméthyl)-L-Tyrosine

On procède comme au stade 1 de l'exemple 16 en utilisant, à la place du L-Tryptophane méthylester, le trifluoroacétate de l'ester méthylique de la O-(phénylméthyl)-L-Tyrosine obtenu à partir de la N-Boc-O-benzyltyrosine L de la façon suivante :

1) par couplage EDC + méthanol (+ HOBT) dans le chlorure de méthylène pour donner l'ester méthylique correspondant
2) par déprotection du Boc avec chlorure de méthylène-acide trifluoroacétique : 50-50 puis 1 h à 0°C et 2 h à

température ambiante et évaporation.

On obtient ainsi le produit attendu.

**Analyses RMN : 1H**

2,4 (s, 3H, SAc) ; 2,6 (m, 1H) ; 2, 8 à 3,2 (m, 7H) ; 3,65 (s, 3H, COOMe) ; 4,8 (q, 1H, CH) ; 5,1 (s, 2H, $CH_2O$) ; 5,85 (d, 1H, NH) ; 6, 9 (d, 2H, Ar) ; 7, 1 (d, 2H, Ar) ; 7,3 à 7,6 (m, 10H).

STADE 2 : (S)N-(2-mercaptométhyl)-1-oxo-3-phénylpropyl)-O-phénylméthyl-L-Tyrosine

On procède comme au stade 4 de l'exemple 10 à partir du produit obtenu au stade 1 ci-dessus et obtient ainsi le produit attendu.

**Analyse :**

SM : $M^+$ = 449
RMN : (δ, $CHCl_3$, ppm) 250 MHz ; 1,37 (m, 1H, SH) ; 2,40 à 3,20 (m, 7H) ; 4, 71 (m, 1H) ; 5,01 (s, 2H, $OCH_2Ph$) ; 5,71 (d, 1H, NH) ; 6,88 (m, 2H) ; 7,0 à 7,4 (m, 12H).

**EXEMPLE 23 : (S)N-(2-(mercaptométhyl)-1-oxo-3-phénylpropyl)-L-Tyrosine**

STADE 1 : ester méthylique de la (S)N-[2-[(acétylthio) méthyl]-1-oxo-3-phénylpropyl]-L-Tyrosine

On introduit 120 mg de produit obtenu au stade 1 de l'exemple 22 dans 1 ml d'éthane thiol, puis ajoute 0,3 ml de $BF_3$ $OEt_2$.
On laisse 3 heures à température ambiante puis verse dans 5 ml d'eau et extrait à l'acétate d'éthyle puis évapore.
Après purification sur silice avec pour éluant acétate d'éthyle-chloroforme : 10-90, on obtient 50 mg de produit attendu.

**Analyses :**

SM : $415^+ = M^+$ ; 238 = $M^+$ - CH ($CO_2Me$) ($CH_2$-phényl-OH) ; $107^+$ : $CH_2$-phényl-OH ; 178 : Me-O-CO-($CH_2$)-phényl-OH.

STADE 2 : (S)N-(2-(mercaptométhyl)-1-oxo-3-phénylpropyl)-L-Tyrosine

On procède comme au stade 4 de l'exemple 10 à partir du produit obtenu au stade 1 ci-dessus et obtient ainsi le produit attendu

**Analyse :**

RMN : (300 MHz δ : DMSO)
1,86 (t, 1H, SH) ; 2,6 à 3,0 (m, 7H) ; 4,38 (m, 1H) ; 6,63 - 7,0 (m, 4H) ; 7,10 - 7,25 (m, 5H) ; 8,20 (d, 1H, NH) ; 9,18 (s, 1H, OH).

**EXEMPLE 24 : Ester méthylique du (R)N-(2-mercapto-1-oxo-3-phénylpropyl) L-Tryptophane**

STADE 1 : ester méthylique du (R)N-[2-[(benzoylthio) méthyl]-1-oxo-3-phénylpropyl]-L-Tryptopbane

On introduit 254 mg de chlorhydrate de l'ester méthylique du L-Tryptophane (commercial) dans 10 ml de dichlorométhane. Après addition de 0,278 mg de triéthylamine, on ajoute 442 mg de BOP puis en 10 minutes une solution de 286 mg d'acide (R) 2-(benzoylthio)-3-phénylpropanoïque (préparé comme indiqué dans la référence J. Org. Chem. 1986, 51, 3664-3671 Bert Strijtveen and Richard M. Kelogg), dans 8 ml de dichlorométhane.
On ajuste ensuite le pH par addition de quelques gouttes de triéthylamine pour le rendre basique et agite à température ambiante, pendant une heure.
La solution organique est ensuite lavée successivement avec acide chlorhydrique 1N, eau déminéralisée,

une solution aqueuse à 10 % de bicarbonate de sodium, eau + eau salée. On sèche, filtre et évapore. Après chromatographie sur silice avec pour éluant chlorure de méthylène-acétate d'éthyle : 95-5, on obtient 211 mg du produit attendu (mousse incolore) isomère RS.

**Analyses :**

$[\alpha]_D$=+83,2°, à la concentration 0,5 dans le méthanol.
IR ($CHCl_3$) ($cm^{-1}$)

| | |
|---|---|
| C=NH | 3479 - 3418 |
| C=O | 1743 - 1678 |
| Aromatiques + hétérocycle + amide II | 1620 - 1597 - 1587 - 1513 - 1497 - 1490 |

STADE 2 : Ester méthylique du (R)N-(2-mercapto-1-oxo-3-phénylpropyl)-L-Tryptophane

On introduit 202 mg du produit obtenu au stade 1 ci-dessus, dans 10 ml de méthanol, refroidie à 20±5°C, ajoute 0,42 ml de soude N et agite 1 heure à cette température.
On acidifie ensuite par addition de 5 ml d'acide chlorhydrique 0,1N, extrait au chlorure de méthylène, lave avec une solution de bicarbonate de sodium, à l'eau et à l'eau salée et évapore à sec. On purifie par chromatographie sur silice avec pour éluant un mélange chlorure de méthylène-acétate d'éthyle : 95-5. On obtient ainsi 94 mg de produit attendu (mousse incolore).
$[\alpha]_D$=+8,16°, à la concentration 0,49 dans le méthanol.

**Analyses physiques :**

Microanalyse :

| | Calculé | Trouvé |
|---|---|---|
| C % | 65,95 | 65,8 |
| H % | 5,80 | 5,9 |
| N % | 7,32 | 7,0 |
| S % | 8,38 | 8,3 |

IR $CHCl_3$ ($cm^{-1}$)

| | |
|---|---|
| =C-NH | 3479 - 3410 - 3306 |
| C=0 | 1741 - 1675 |
| Hétérocycle + Aromatiques + Amide II | 1620 - 1604 - 1584 - 1512 - 1498 |
| SH | 2534 |

**EXEMPLE 25 : (S)N-(2-mercapto-1-oxo-3-phénylpropyl)D-Tryptophane**

STADE 1 : ester méthylique du (S)N-[2-[(benzoylthio) méthyl]-1-oxo-3-phénylpropyl]-D-Tryptophane

On procède comme au stade 1 de l'exemple 24 en utilisant, au lieu du chlorhydrate de l'ester méthylique de

L-Tryptophane, le chlorhydrate de l'ester méthylique de D-Tryptophane et, au lieu de l'acide (R)-2-(benzoylthio)-3-phénylpropanoïque, l'acide (S)-2-(benzoylthio)-3-phénylpropanoïque (préparé selon la même référence). Après purification par chromatographie sur silice avec pour éluant le mélange chlorure de méthylène-acétate d'éthyle : 95-5, on obtient 564 mg de produit attendu (isomère SR) (mousse incolore). $[\alpha]_D$=+85,09°, à la concentration 0,5 dans le méthanol.

**Analyses**

IR ($CHCl_3$) $cm^{-1}$

| | |
|---|---|
| =C-NH | 3480 |
| C=O | 1743 - 1678 |
| Aromatique + Système conjugué + Amide II | 1620 - 1598 - 1582 - 1514 - 1497 - 1490 |

STADE 2 : ester méthylique du (S)N-[2-mercapto-1-oxo-3-phénylpropyl]-D-Tryptophane

On procède comme au stade 2 de l'exemple 24 à partir de 564 mg du produit obtenu au stade 1 ci-dessus puis par acidification par addition de 15 ml d'acide chlorhydrique 0,1N. On obtient ainsi 378 mg de produit attendu (mousse blanche solide).
$[\alpha]_D$=+8,30°, à la concentration de 0,53 % de méthanol.

**Analyses :**

IR ($CHCl_3$) ($cm^{-1}$)

| | |
|---|---|
| =C-NH | 3480 - 3421 |
| >=O | 1740 - 1675 |
| Système conjugué + Aromatique + Amide II | 1618 - 1600 - 1585 - 1512 - 1498 |
| SH | 2570 |

STADE 3 : (S)N-(2-mercapto-1-oxo-3-phénylpropyl)-D-Tryptophane

On procède comme au stade 4 de l'exemple 10 à partir de 363 mg du produit obtenu au stade 2 ci-dessus dans 15 ml de mélange tétrahydrofuranne-eau 2-1 et 63 mg de lithine monohydratée. On obtient ainsi 184 mg de produit attendu (mousse solide blanche).
$[\alpha]_D$=-7,2°, à la concentration de 0,5 de méthanol

**Analyses :**

Microanalyse :

| | Calculé | Trouvé |
|---|---|---|
| C % | 65,20 | 65,4 |
| H % | 5,47 | 5,5 |
| N % | 7,60 | 7,3 |
| S % | 8,70 | 8,3 |

IR $CHCl_3$ ($cm^{-1}$)

| | |
|---|---|
| =C-NH | 3478 - 3418 |
| + absorption générale NH/OH | |
| C=0 | 1722 - 1672 |
| Hétérocycle + Aromatiques + Amide II | 1621 - 1604 - 1583 - 1513 - 1497 |
| SH | 2570 |

**EXEMPLE 26 : (R)N-(2-mercapto-1-oxo-3-phénylpropyl)-L-Tryptophane**

On procède comme au stade 4 de l'exemple 10 à partir de 130 mg du produit de l'exemple 24, 28 mg d'hydroxyde de lithium, eau et 5 ml d'un mélange tétrahydrofuranne-eau : 2/1. On obtient ainsi 120 mg de produit attendu.

**Analyses :**

IR $CHCl_3$ ($cm^{-1}$)

| | |
|---|---|
| =C-NH | 3478 - 3415 |
| absorption générale OH/NH | |
| C=0 | 1722 - 1672 |
| Hétérocycle + Aromatiques + Amide II | 1621 - 1604 - 1583 - 1498 |
| SH | 2574 |

**EXEMPLE 27 : (S)N-(2-mercapto-1-oxo-3-phénylpropyl)-L-Tryptophane**

STADE 1 : ester méthylique du (S)N-[2-[(benzoylthio) méthyl]-1-oxo-3-phénylpropyl]-L-Tryptophane

On procède comme au stade 1 de l'exemple 24 en utilisant, au lieu de l'acide (R)-2-(benzoylthio)-3-phénylpropanoïque, l'acide (S)-2-(benzoylthio)-3-phénylpropanoïque, et de même l'ester méthylique du L-Tryptophane. Après chromatographie sur silice avec pour éluant le mélange chlorure de méthylène-acétate d'éthyle : 95-5, on isole 412 mg du produit attendu SS (mousse incolore).
$[\alpha]_D$=-68°, à la concentration 0,5 dans le méthanol.

Analyses

IR ($CHCl_3$) $cm^{-1}$

| | |
|---|---|
| =C-NH | 3479 |
| C=0 | 1742 - 1676 |
| Hétérocycle + Aromatiques + Amide II | 1620 - 1603 - 1582 - 1514 - 1498 - 1490 |

STADE 2 : ester méthylique du (S)N-[2-mercapto-1-oxo-3-phénylpropyl]-L-Tryptophane

On procède comme au stade 2 de l'exemple 24, à partir de 496 mg du produit obtenu au stade 1 ci-dessus, dans 15 ml de méthanol et 1,07 ml de soude N. On obtient ainsi 272 mg de produit attendu. F = 117-119°C. $[\alpha]_D$=+24,8°, à la concentration 0,5 dans le méthanol.

**Analyses**

IR ($CHCl_3$) $cm^{-1}$

| | |
|---|---|
| -NH | 3479 - 3414 - 3370 |
| >=0 | 1742 - 1667 |
| Hétérocycle + Aromatiques + Amide | 1620 - 1602 - 1580 - 1512 - 1498 |

STADE 3 : (S)N-(2-mercapto-1-oxo-3-phénylpropyl)-L-Tryptophane

On procède comme au stade 4 de l'exemple 10 à partir de 249 mg du produit obtenu au stade 2 ci-dessus, dans 15 ml d'un mélange tétrahydrofuranne-eau : 2-1 et 60 mg de lithine monohydratée.

On obtient ainsi 175 mg de produit attendu (mousse amorphe).
$[\alpha]_D$=+28,8°, à la concentration 0,5 dans le méthanol.

**Analyses :**

Microanalyse :

| | Calculé | Trouvé |
|---|---|---|
| C % | 65,20 | 65,8 |
| H % | 5,47 | 5,6 |
| N % | 7,60 | 7,2 |
| S % | 8,70 | 8,1 |

IR $CHCl_3$ ($cm^{-1}$)

| | |
|---|---|
| -NH | 3478 - 3408 - 3375 |
| Absorption générale OH/NH | |
| >=0 | 1743 - 1721 - 1667 |
| Système conjugué Aromatiques + Amide II | 1604 - 1586 - 1513 - 1497 |

**ENSEMBLE 29 DE COMPOSITION PHARMACEUTIQUE :**

On a préparé des comprimés répondant à la formule suivante :

| | |
|---|---|
| Produit de l'exemple 4 | 50 mg |
| Excipient pour un comprimé terminé à | 200 mg |

(détail de l'excipient : lactose, talc, amidon, stéarate de magnésium).

## RESULTATS PHARMACOLOGIQUES

### Détermination de l'effet inhibiteur de l'enzyme de conversion de l'endothéline (ECE)

On utilise un test, dans lequel le produit (2,3-$^3$H)propionyl-b-ET-1(19-35) préparé comme indiqué ci-dessous en a) est clivé par l'ECE en le produit (2,3-$^3$H)propionyl-b-ET-1(19-21) en présence du produit P dont on veut déterminer l'activité inhibitrice de l'ECE comme indiqué ci-dessous en b) : l'activité inhibitrice de l'ECE du produit P sera donc d'autant plus grande que la quantité de produit de formule (II) formé, déterminée par comptage de la radioactivité, sera plus faible. On procède comme suit :

### a) - Préparation du peptide tritié (2,3-$^3$H)propionyl-b-ET-1 (19-35)

Le N-succinimidyl-(2,3-$^3$H)-propionate (Amersham code TRK.556) est en solution dans 5 ml de toluène à 1 mCi/ml, 99 mCi/mmol ==> 5 mCi et 50 nmoles.

On évapore le toluène jusqu'à obtenir une solution de 10 µl puis ajoute 25 µl d'une solution de DMSO contenant 0,2 mg de b-ET-1(19-35), la b-ET-1(19-35) (MW-2014,2) ayant été préalablement séchée sur potasse une nuit. On continue à évaporer durant 10 mn puis agite la solution sous courant d'azote pendant 15 mn afin d'éliminer le toluène résiduel.

On agite alors doucement durant 4 jours.

Pour purifier, on ajoute 225 µl de tampon phosphate pH 6,5 et 50 µl d'acétonitrile puis agite 10 mn et la solution radioactive obtenue est séparée en 2 injections de 150 µl sur une colonne de Nucléosil $C_{18}$ (150 x 4,6 mm).

L'élution est effectuée avec un débit de 0,8 ml/mn par un gradient de 0 à 20 % d'acétonitrile en 20 mn puis 20 à 35 % en 50 mn.

Analyses :

L'analyse des fractions est effectuée par comptage du tritium au compteur à scintillation liquide (1 µl dans 5 ml de scintillant HiSafe3) durant 60 secondes.

Les fractions radioactives sont réunies et fractionnées en échantillons de 200 µl dans des tubes eppendorf siliconnés que l'on peut conserver à -80°C ou -20°C.

Caractéristiques du produit obtenu :

dpm = 1 067 274 560 correspondant à 0,5 mCi.
Rendement radioactif : 10 %.
Activité spécifique : (99 x 10)/30,5 = 32,5 Ci/mmol.

### b) - Détermination de l'activité inhibitrice de l'enzyme de conversion de l'endothéline (ECE)

10 µl d'ECE soit 1 à 2 µg d'ECE purifié sont pré-incubés pendant 30 minutes à 37°C, dans 400 µl d'un tampon de Tris maléate 50 mM pH = 6,5, 20 µl de chlorure de sodium 5M et 5 µl de produit P dont on veut tester l'activité inhibitrice, en solution à différentes concentrations comprises entre 1 µM à 100 mM (soit des concentrations finales en produit P de 10 nM à 1 mM).

La réaction est initiée par addition de 10 µl de (2,3-$^3$H)propionyl-b-ET-1(19-35) préparé comme indiqué ci-dessus en a), à la concentration finale de $1,8.10^{-12}$M.

Après une heure d'incubation à 37°C, la réaction est arrêtée par addition de 600 µl d'acétate d'éthyle et le (2,3-$^3$H)propionyl-b-ET-1(19-21) est extrait par agitation mécanique pendant 2 minutes.

On prélève 300 µl de la phase organique et ajoute 5 ml de liquide scintillant et compte la radioactivité pendant 1 minute au compteur à scintillation liquide.

Chaque mesure est réalisée en triplicate excepté pour le témoin d'ECE (témoin enzyme, sans produit dont on veut tester l'activité inhibitrice de l'ECE), pour lequel la mesure est réalisée en sextuplet.

Le pourcentage d'inhibition est calculé en faisant le rapport :

$$\frac{\text{produit testé - blanc}}{\text{témoin enzyme - blanc}}$$

Le blanc est effectué à partir de la solution obtenue sans enzyme.
Le test a été validé par son application à des inhibiteurs connus soit P1 qui est le phosphoramidon et P2 qui est le N-(phényléthylphosphonyl)-Leu-Trp (TAKEDA).
Le tableau ci-après donne les résultats obtenus en utilisant comme produits P des produits en exemples dans la présente invention, dont on veut tester les activités inhibitrices de l'ECE.
A partir des cpm obtenus et par tracé du graphique du pourcentage d'inhibition par rapport à la concentration en inhibiteur (nM), on calcule la $CI_{50}$ qui correspond donc à la concentration provoquant une inhibition de 50 % de l'ECE.
Les résultats chiffrés obtenus sont indiqués dans le tableau ci-dessous :
RESULTATS :

| Produits des exemples | $CI_{50}$ (nM) |
|---|---|
| 1 | 20 |
| 2 | 40 |
| 4 | 25 |
| 5 | 40 |
| 7A | 120 |
| 7B | 80 |
| 8 | 150 |
| 9 | 70 |
| 10 | 55 |
| 17 | 180 |
| 21 | 150 |

## Revendications

1. Utilisation pour la préparation de médicaments destinés au traitement de pathologies requérant une inhibition de l'enzyme de conversion de l'endothéline, ces pathologies étant les spasmes vasculaires, le vasospasme consécutif à une hémorragie cérébrale, les spasmes coronariens, les spasmes vasculaires périphériques, les insuffisances rénales, l'infarctus du myocarde, l'insuffisance cardiaque congestive, l'athéro-sclérose, l'hypertension pulmonaire, l'asthme, l'ostéo-porose, l'hypertrophie prostatique, l'hypertension induite par l'endothéline, ainsi que la prévention des resténoses post-angioplastie et des fibroses cardiaques et vasculaires, de produits de formule (I) :

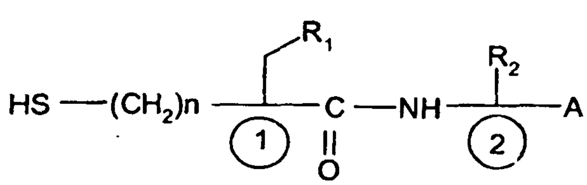

(I)

dans laquelle :

n représente l'entier 0 ou 1,
$R_1$ représente un radical phényle ou biphényle éventuellement substitué par un ou plusieurs radicaux choisis parmi les atomes d'halogène, les radicaux hydroxyle éventuellement protégé, alcoxy linéaire ou ramifié renfermant au plus 4 atomes de carbone, phénoxy, cyano, carboxy libre, salifié, estérifié ou amidifié, benzyloxy et le radical dioxol,

$R_2$ représente un radical méthyle substitué par un radical phényle, phénylthio ou indolyle et éventuellement par un deuxième radical phényle, ces radicaux phényle, phénylthio et indolyle étant éventuellement substitués par un ou plusieurs radicaux choisis parmi les atomes d'halogène, les radicaux hydroxyle éventuellement protégé, alcoxy linéaire ou ramifié renfermant au plus 4 atomes de carbone, cyano, carboxy libre, salifié, estérifié ou amidifié, benzyloxy, thiényle, naphtyle et phényle, ces trois derniers radicaux étant eux-mêmes éventuellement substitués par un ou plusieurs radicaux choisis parmi les atomes d'halogène, les radicaux hydroxyle éventuellement protégé, alcoxy linéaire ou ramifié renfermant au plus 4 atomes de carbone, cyano et carboxy libre, salifié, estérifié ou amidifié,
A représente le radical carboxy libre, salifié, estérifié ou amidifié, le radical tétrazolyle libre ou salifié, ou un radical alkyle, renfermant au plus 10 atomes de carbone et substitué par un radical choisi parmi les radicaux carboxy libre, salifié, estérifié ou amidifié, les radicaux hydroxyle éventuellement protégé, alcoxy renfermant au plus 4 atomes de carbone, phénoxy, phényle, naphtyle, thiényle, indolyle et pyridyle, ces radicaux étant éventuellement substitués par un ou plusieurs radicaux choisis parmi les atomes d'halogène, les radicaux hydroxyle éventuellement protégé, alcoxy linéaire ou ramifié renfermant au plus 4 atomes de carbone, cyano et carboxy libre, salifié, estérifié ou amidifié,
① et ② indiquant les centres, le cas échéant, asymétriques des produits de formule (I),

lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I), à l'exclusion du thiorphan.

**2.** Utilisation telle que définie à la revendication 1, dans laquelle :

n représente l'entier 0 ou 1,
$R_1$ représente un radical phényle ou biphényle éventuellement substitué par un ou deux radicaux choisis parmi les atomes d'halogène, les radicaux hydroxyle éventuellement protégé, alcoxy linéaire ou ramifié renfermant au plus 4 atomes de carbone, benzyloxy et le radical dioxol,
$R_2$ représente un radical méthyle substitué par un radical phényle, phénylthio ou indolyle et éventuellement par un deuxième radical phényle, ces radicaux phényle, phénylthio et indolyle étant éventuellement substitués par un radical choisi parmi les radicaux hydroxyle éventuellement protégé, alcoxy linéaire ou ramifié renfermant au plus 4 atomes de carbone, benzyloxy, thiényle, naphtyle et phényle lui-même éventuellement substitué par un radical hydroxyle éventuellement protégé ou alcoxy linéaire ou ramifié renfermant au plus 4 atomes de carbone,
et A représente le radical carboxy libre, salifié, estérifié ou amidifié, tétrazolyle libre ou salifié, ou un radical alkyle, renfermant au plus 8 atomes de carbone et substitué par un radical choisi parmi les radicaux carboxy libre, salifié, estérifié ou amidifié, les radicaux hydroxyle éventuellement protégé, alcoxy renfermant au plus 4 atomes de carbone, et phénoxy,

lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

**3.** Utilisation telle que définie à la revendication 1 ou 2, dans laquelle n, $R_2$ et A ont les significations indiquées à la revendication 1 ou 2,
et $R_1$ représente un radical phényle ou biphényle, éventuellement substitué soit par un atome de brome ou un radical benzyloxy, soit par un radical dioxol et éventuellement un atome d'halogène,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

**4.** Utilisation telle que définie aux revendications 1 à 3, dans laquelle :

n représente l'entier 1,
$R_1$ représente un radical phényle ou biphényle, éventuellement substitué soit par un atome de brome ou un radical benzyloxy, soit par un radical dioxol et éventuellement un atome d'halogène,
$R_2$ représente un radical méthyle substitué soit par un radical indolyle lui-même éventuellement substitué par un radical benzyloxy, soit par deux radicaux phényle, soit par un radical phénylthio ou phényle, lui-même éventuellement substitué par un radical thiényle, naphtyle ou phényle lui-même éventuellement substitué par

un radical hydroxyle éventuellement protégé, alcoxy renfermant au plus 4 atomes de carbone ou benzyloxy, A représente le radical carboxy libre, salifié, estérifié ou amidifié ou un radical alkyle, renfermant au plus 8 atomes de carbone substitué par un radical phénoxy,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

**5.** Utilisation telle que définie à la revendication 1, des produits dont les noms suivent :

- N-[3-(3-bromophényl)-2-(mercaptométhyl)-1-oxopropyl]-L-Tryptophane,
- N-(3-((1,1'-biphényl)-3-yl)-2-(mercaptométhyl)-1-oxopropyl]-L-Tryptophane,
- (R,S) N-(3-(4-bromophényl)-2-(mercaptométhyl)-1-oxopropyl] L-Tryptophane,
- (R,S) N-(3-((1,1'-biphényl)-4-yl)-2-(mercaptométhyl)-1-oxopropyl]-L-Tryptophane,
- (S) N-[2-(mercaptométhyl)-1-oxo-3-phénylpropyl]-4-(2-thiényl)-L-Phénylalanine,
- (S) N-[2-(mercaptométhyl)-1-oxo-3-phénylpropyl]-4-(1-naphtyl)-L-Phénylalanine,
- (S) N-(2-(mercaptométhyl) 1-oxo-3-phénylpropyl] L-Tryptophane,
- (S) 4-phényl-N-((2-mercaptométhyl)-1-oxo-3-phénylpropyl]-L-Phénylalanine,
- (S, S) N-(2-((1,1'-biphényl)-4-yl)-1-(1H-tétrazol-5-yl)-éthyl)-alpha-(mercaptométhyl)-benzènepropanamide,
- (R) et (S) N-[3-(3-bromophényl)-2-(mercaptométhyl)-1-oxopropyl]-4-(2-thiényl)-L-Phénylalanine (isomères A et B),
- N-[3-(3-bromophényl)-2-(mercaptométhyl)-1-oxopropyl]-7-(phénylméthoxy)-Tryptophane,

lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

**6.** Procédé de préparation des produits de formule (I) pour l'utilisation telle que définie à la revendication 1, **caractérisé en ce que soit** l'on soumet un produit de formule (II) :

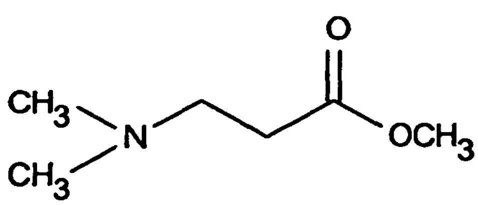

(II)

à l'action d'un produit de formule (III) :

$$R'_1-CH_2-Hal_1 \quad (III)$$

dans laquelle $Hal_1$ représente un atome de chlore ou d'iode, $R'_1$ a la signification indiquée à la revendication 1 pour $R_1$ dans laquelle les éventuelles fonctions réactives sont éventuellement protégées, pour obtenir le produit de formule (IV) :

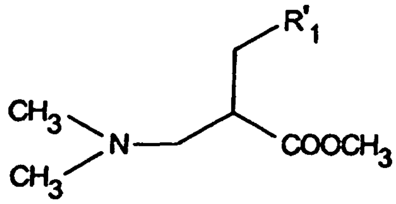

(IV)

dans laquelle $R'_1$ a la signification indiquée ci-dessus,

que l'on soumet à l'action de l'iodure de méthyle pour obtenir le produit de formule (V) :

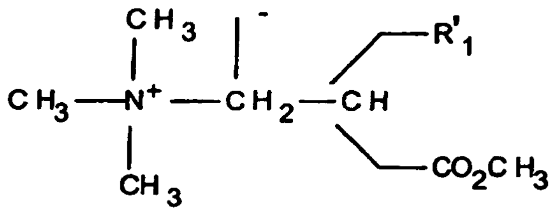

(V)

dans laquelle $R'_1$ a la signification indiquée ci-dessus, que l'on soumet à une réaction d'élimination et de saponification, pour obtenir le produit de formule (VI) :

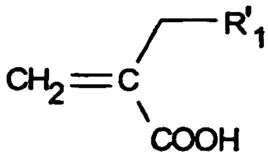

(VI)

dans laquelle $R'_1$ a la signification indiquée ci-dessus
pour $R_1$, que l'on soumet à l'action du composé de formule (XIIa)

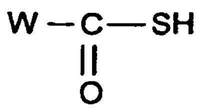

(XIIa)

dans lequel W représente un radical alkyle ou phényle, pour obtenir le produit de formule (VII) :

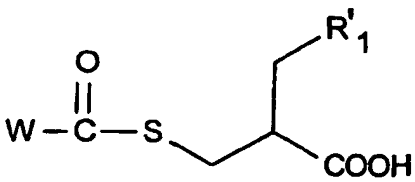

(VII)

dans laquelle $R'_1$ et W ont les significations indiquées ci-dessus, que l'on soumet :

ou bien à l'action, en présence d'un agent couplant, d'un produit de formule (VIII) :

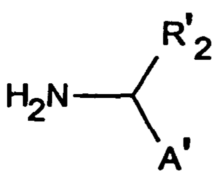

(VIII)

dans laquelle $R'_2$ a la signification indiquée à la revendication 1 pour $R_2$ dans laquelle les éventuelles fonctions réactives sont éventuellement protégées, et A' a la signification indiquée à la revendication 1 pour A, dans laquelle les éventuelles fonctions réactives sont éventuellement protégées, pour obtenir le produit de formule (IX) :

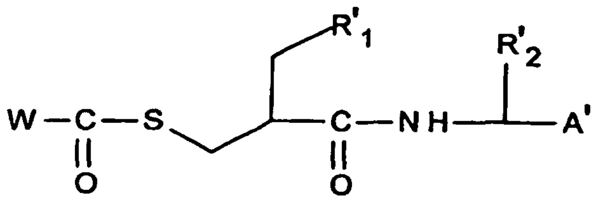

(IX)

dans laquelle R'$_1$, R'$_2$, W et A' ont les significations indiquées ci-dessus,
ou bien à l'action d'un agent chlorant pour obtenir le chlorure d'acide correspondant, que l'on fait réagir avec le composé de formule (VIII) telle que définie ci-dessus, pour obtenir le produit de formule (IX) tel que définie ci-dessus,
**soit** l'on soumet un produit de formule (X) :

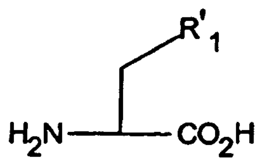

(X)

dans laquelle R'$_1$ a la signification indiquée ci-dessus, à une réaction de diazotation en présence d'un agent halogénant, pour obtenir le produit de formule (XI) :

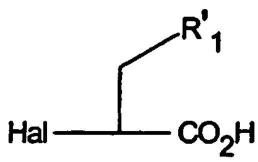

(XI)

dans laquelle R'$_1$ a la signification indiquée ci-dessus et Hal représente un atome d'halogène, que l'on soumet à l'action du thioacétate de formule (XIIb) :

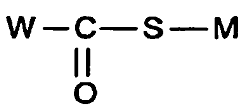

(XIIb)

dans laquelle M représente un métal alcalin et W a la signification indiquée ci-dessus, pour obtenir le produit de formule (XIII) :

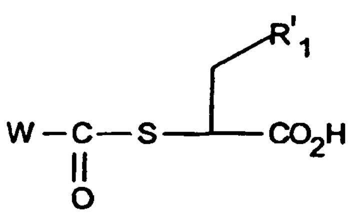

(XIII)

dans laquelle W et R'$_1$ ont les significations indiquées ci-dessus, que l'on soumet à l'action du produit de formule (VIII) telle que définie ci-dessus, pour obtenir le produit de formule (XIV) :

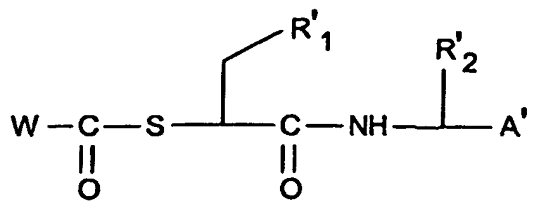

(XIV)

dans laquelle $R'_1$, $R'_2$, A' et W ont les significations indiquées ci-dessus,
produits de formules (IX) et (XIV) que, pour obtenir des produits de formule (I) ou pour transformer des produits de formule (I) en d'autres produits de formule (I), l'on peut traiter, si désiré et si nécessaire, à l'une ou plusieurs des réactions suivantes, dans un ordre quelconque :

- une réaction de saponification de fonction ester en fonction acide,
- une réaction de transformation de fonction cyano en fonction acide ou tétrazolyle,
- une réaction de transformation de fonction alcoxy en fonction hydroxyle,
- une réaction d'estérification, salification ou amidification de fonction acide,
- une réaction de libération de la fonction thiol à partir du radical

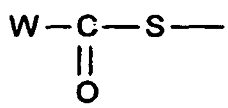

- une réaction d'élimination des groupements protecteurs que peuvent porter les fonctions réactives protégées,
- une réaction de salification par un acide ou une base minéral(e) ou organique pour obtenir le sel correspondant,

lesdits produits de formule (I) ainsi obtenus étant sous toutes les formes isomères possibles racémiques, énantiomères et diastéréoisomères.

**7.** Les produits suivants :

- N-[3-(3-bromophényl)-2-(mercaptométhyl)-1-oxopropyl]-L-Tryptophane,
- N-(3-((1,1'-biphényl)-3-yl)-2-(mercaptométhyl)-1-oxopropyl]-L-Tryptophane,
- (R,S) N-(3-(4-bromophényl)-2-(mercaptométhyl)-1-oxopropyl] L-Tryptophane,
- (R,S) N-(3-((1,1'-biphényl)-4-yl)-2-(mercaptométhyl)-1-oxopropyl]-L-Tryptophane,
- (S) N-[2-(mercaptométhyl)-1-oxo-3-phénylpropyl]-4-(2-thiényl)-L-Phénylalanine,
- (S) N-[2-(mercaptométhyl)-1-oxo-3-phénylpropyl]-4-(1-naphtyl)-L-Phénylalanine,
- (S) 4-phényl-N-((2-mercaptométhyl)-1-oxo-3-phénylpropyl]-L-Phénylalanine,
- (S,S) N-(2-((1,1'-biphényl)-4-yl)-1-(1H-tétrazol-5-yl)-éthyl)-alpha-(mercaptométhyl)-benzènepropanamide,
- (R) et (S) N-[3-(3-bromophényl)-2-(mercaptométhyl)-1-oxopropyl]-4-(2-thiényl)-L-Phénylalanine (isomères A et B),
- N-[3-(3-bromophényl)-2-(mercaptométhyl)-1-oxopropyl]-7 (phénylméthoxy)-Tryptophane,

lesdits produits étant sous toutes les formes isomères racémiques, énantiomères et diastéréoisomères possibles, ainsi que les sels d'addition avec les acides minéraux et organiques ou avec les bases minérales et organiques pharmaceutiquement acceptables, desdits produits de formule(I).

**8.** Les compositions pharmaceutiques contenant, à titre de principe actif, l'un au moins des produits tels que définis à la revendication 7.

## Patentansprüche

**1.** Verwendung für die Herstellung von Medikamenten, die zur Behandlung von Krankheiten, die eine Hemmung des Endothelin-Konversionsenzyms erfordern, wobei diese Krankheiten Gefäßspasmen, Gefäßspasmus als Folge einer Himblutung, Koronarspasmen, periphäre Gefäßspasmen, Niereninsuffizienzen, Myokardinfarkt, kongestive Herzinsuffizienz, Atherosklerose, Lungenhochdruck, Asthma, Osteoporose, Prostatahypertrophie, von Endothelin bewirkter Hochdruck sind, sowie zur Vorbeugung von Restenosen nach Angoplastie und von Herz- und Gefäßfibrosen bestimmt sind, von Produkten der Formel (I):

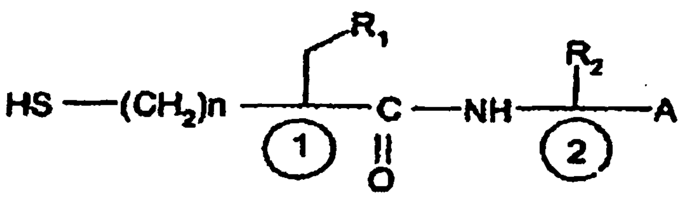

(I)

worin:

n die ganze Zahl 0 oder 1 darstellt,
$R_1$ einen Phenyl- oder Biphenylrest darstellt, der gegebenenfalls mit einem oder mehreren Resten substituiert ist, die ausgewählt sind unter den Halogenatomen, den Resten Hydroxyl, gegebenenfalls geschützt, Alkoxy, linear oder verzweigt, mit höchstens 4 Kohlenstoffatomen, Phenoxy, Cyano, Carboxy in freier Form, Salz-, Ester- oder Amidform, Benzyloxy und dem Dioxolrest,
$R_2$ einen Methylrest darstellt, der mit einem Phenyl-, Phenylthio- oder Indolylrest und gegebenenfalls mit einem zweiten Phenylrest substituiert ist, wobei diese Phenyl-, Phenylthio- und Indolylreste gegebenenfalls mit einem oder mehreren Resten substituiert sind, die ausgewählt sind unter den Halogenatomen, den Resten Hydroxyl, gegebenenfalls geschützt, Alkoxy, linear oder verzweigt, mit höchstens 4 Kohlenstoffatomen, Cyano, Carboxy in freier Form, Salz-, Ester- oder Amidform, Benzyloxy, Thienyl, Naphthyl und Phenyl, wobei diese drei letzten Reste gegebenenfalls selbst mit einem oder mehreren Resten substituiert sind, die ausgewählt sind unter den Halogenatomen, den Resten Hydroxyl, gegebenenfalls geschützt, Alkoxy, linear oder verzweigt, mit höchstens 4 Kohlenstoffatomen, Cyano und Carboxy in freier Form, Salz-, Ester- oder Amidform,
A den Carboxyrest in freier Form, Salz-, Ester- oder Amidform, den Tetrazolylrest in freier Form oder in Salzform oder einen Alkylrest darstellt, der höchstens 10 Kohlenstoffatome enthält und mit einem Rest substituiert ist, der ausgewählt ist unter den Carboxyresten in freier Form, Salz-, Ester- oder Amidform, den Resten Hydroxyl, gegebenenfalls geschützt, Alkoxy mit höchstens 4 Kohlenstoffatomen, Phenoxy, Phenyl, Naphthyl, Thienyl, Indolyl und Pyridyl, wobei diese Reste gegebenenfalls mit einem oder mehreren Resten substituiert sind, die ausgewählt sind unter den Halogenatomen, den Resten Hydroxyl, gegebenenfalls geschützt, Alkoxy, linear oder verzweigt, mit höchstens 4 Kohlenstoffatomen, Cyano und Carboxy in freier Form, Salz-, Ester- oder Amidform,

wobei ① und ② die gegebenenfalls asymmetrischen Zentren der Produkte der Formel (I) darstellen, und besagte Produkte der Formel (I) in allen möglichen racemischen, enantiomeren und diastereoisomeren Isomerformen vorkommen, sowie der Additionssalze mit den anorganischen und organischen Säuren oder mit den anorganischen oder organischen Basen besagter Produkte der Formel (I) mit Ausnahme von Thiorphan.

**2.** Verwendung, wie in Anspruch 1 definiert, bei der:

n die ganze Zahl 0 oder 1 darstellt,
$R_1$ einen Phenyl- oder Biphenylrest darstellt, der gegebenenfalls mit einem oder zwei Resten substituiert ist, die ausgewählt sind unter den Halogenatomen, den Resten Hydroxyl, gegebenenfalls geschützt, Alkoxy, linear oder verzweigt, mit höchstens 4 Kohlenstoffatomen, Benzyloxy und dem Dioxolrest,
$R_2$ einen Methylrest darstellt, der mit einem Phenyl-, Phenylthio- oder Indolylrest und gegebenenfalls mit einem zweiten Phenylrest substituiert ist, wobei diese Phenyl-, Phenylthio- und Indolylreste gegebenenfalls mit einem Rest substituiert sind, der ausgewählt ist unter den Resten Hydroxyl, gegebenenfalls geschützt, Alkoxy, linear oder verzweigt, mit höchstens 4 Kohlenstoffatomen, Benzyloxy, Thienyl, Naphthyl und Phenyl, der selbst gegebenenfalls mit einem Rest Hydroxyl, gegebenenfalls geschützt, oder Alkoxy, linear oder verzweigt, mit höchstens 4 Kohlenstoffatomen substituiert ist,
und A den Carboxyrest in freier Form, Salz-, Ester- oder Amidform, Tetrazolylrest in freier Form oder Salzform oder einen Alkylrest darstellt, der höchstens 8 Kohlenstoffatome enthält und mit einem Rest substituiert ist, der ausgewählt ist unter den Carboxyresten in freier Form, Salz-, Ester- oder Amidform, den Resten Hydroxyl,

gegebenenfalls geschützt, Alkoxy mit höchstens 4 Kohlenstoffatomen und Phenoxy, wobei besagte Produkte der Formel (I) in allen möglichen racemischen, enantiomeren und diastereoisomeren Isomerformen vorkommen, sowie der Additionssalze mit den anorganischen und organischen Säuren oder mit den anorganischen oder organischen Basen besagter Produkte der Formel (I).

**3.** Verwendung, wie in Anspruch 1 oder 2 definiert, bei der n, $R_2$ und A die in Anspruch 1 oder 2 angegebenen Bedeutungen haben,
und $R_1$ einen Phenyl- oder Biphenylrest darstellt, der gegebenenfalls entweder mit einem Bromatom oder einem Benzyloxyrest oder mit einem Dioxolrest und gegebenenfalls einem Halogenatom substituiert ist,
wobei besagte Produkte der Formel (I) in allen möglichen racemischen, enantiomeren und diastereoisomeren Isomerformen vorkommen, sowie der Additionssalze mit den anorganischen und organischen Säuren oder mit den anorganischen oder organischen Basen besagter Produkte der Formel (I).

**4.** Verwendung, wie in den Ansprüchen 1 bis 3 definiert, bei der:

n die ganze Zahl 1 darstellt,
$R_1$ einen Phenyl- oder Biphenylrest darstellt, der gegebenenfalls entweder mit einem Bromatom oder einem Benzyloxyrest oder mit einem Dioxolrest und gegebenenfalls einem Halogenatom substituiert ist,
$R_2$ einen Methylrest darstellt, der entweder mit einem Indolylrest, der selbst gegebenenfalls mit einem Benzyloxyrest substituiert ist, oder mit zwei Phenylresten oder mit einem Phenylthio- oder Phenylrest substituiert ist, der selbst gegebenenfalls substituiert ist mit einem Thienyl-, Naphthyl- oder Phenylrest, der selbst gegebenenfalls substituiert ist mit einem Rest Hydroxyl, gegebenenfalls geschützt, Alkoxy mit höchstens 4 Kohlenstoffatomen oder Benzyloxy,
A den Carboxyrest in freier Form, Salz-, Ester- oder Amidform oder einen Alkylrest mit höchstens 8 Kohlenstoffatomen, der mit einem Phenoxyrest substituiert ist, darstellt,

wobei besagte Produkte der Formel (I) in allen möglichen racemischen, enantiomeren und diastereoisomeren Isomerformen vorkommen, sowie der Additionssalze mit den anorganischen und organischen Säuren oder mit den anorganischen oder organischen Basen besagter Produkte der Formel (I).

**5.** Verwendung, wie in Anspruch 1 definiert, der Produkte, deren Namen folgen:

- N-[3-(3-Bromphenyl)-2-(mercaptomethyl)-1-oxopropyl]-L-Tryptophan,
- N-[3-((1,1'-Biphenyl)-3-yl)-2-(mercaptomethyl)-1-oxopropyl]-L-Tryptophan,
- (R,S)-N-[3-(4-Bromphenyl)-2-(mercaptomethyl)-1-oxopropyl]-L-Tryptophan,
- (R,S)-N-[3-((1,1'-Biphenyl)-4-yl)-2-(mercaptomethyl)-1-oxopropyl]-L-Tryptophan,
- (S)-N-[2-(Mercaptomethyl)-1-oxo-3-phenylpropyl]-4-(2-thienyl)-L-Phenylalanin,
- (S)-N-[2-(Mercaptomethyl)-1-oxo-3-phenylpropyl]-4-(1-naphthyl)-L-Phenylalanin,
- (S)-N-[2-(Mercaptomethyl)-1-oxo-3-phenylpropyl]-L-Tryptophan,
- (S)-4-Phenyl-N-[2-(mercaptomethyl)-1-oxo-3-phenylpropyl]-L-Phenylalanin,
- (S,S)-N-(2-((1,1'-Biphenyl)-4-yl)-1-(1H-tetrazol-5-yl)-ethyl)-alpha-(mercaptomethyl)-benzenpropanamid,
- (R)- und (S)-N-[3-(3-Bromphenyl)-2-mercaptomethyl)-1-oxopropyl]-4-(2-thienyl)-L-Phenylalanin (Isomere A und B),
- N-[3-(3-Bromphenyl)-2-(mercaptomethyl)-1-oxopropyl]-7-(phenylmethoxy)-Tryptophan,

wobei besagte Produkte der Formel (I) in allen möglichen racemischen, enantiomeren und diastereoisomeren Isomerformen vorkommen, sowie der Additionssalze mit den anorganischen und organischen Säuren oder mit den anorganischen oder organischen Basen besagter Produkte der Formel (I).

**6.** Verfahren zur Herstellung der Produkte der Formel (I) für die Verwendung, wie in Anspruch 1 definiert, **dadurch gekennzeichnet, dass** man entweder ein Produkt der Formel (II):

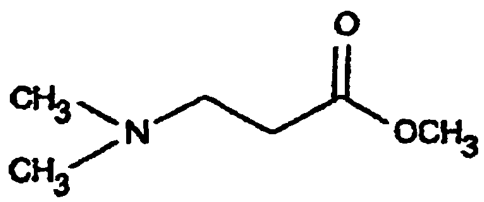

(II)

der Einwirkung eines Produkts der Formel (III):

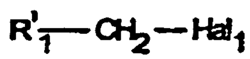

(III)

worin $Hal_1$ ein Chlor- oder Iodatom darstellt, $R'_1$ die in Anspruch 1 für $R_1$ angegebene Bedeutung hat, worin die eventuellen reaktiven Funktionen gegebenenfalls geschützt sind, unterzieht, um das Produkt der Formel (IV):

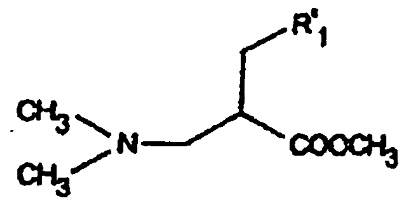

(IV)

worin $R'_1$ die oben angegebene Bedeutung hat, zu erhalten, das man der Einwirkung von Methyliodid unterzieht, um das Produkt der Formel (V):

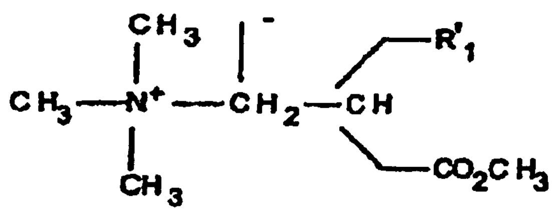

(V)

worin $R'_1$ die oben angegebene Bedeutung hat, zu erhalten, das man einer Eliminierungs- und Verseifungsreaktion unterzieht, um das Produkt der Formel (VI):

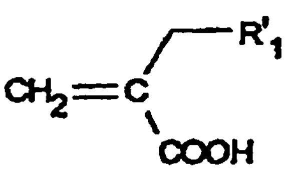

(VI)

worin $R'_1$ die oben für $R_1$ angegebene Bedeutung hat, zu erhalten, das man der Einwirkung der Verbindung der Formel (XIIa):

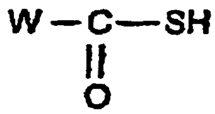

(XIIa)

worin W einen Alkyl- oder Phenylrest darstellt, unterzieht, um das Produkt der Formel (VII):

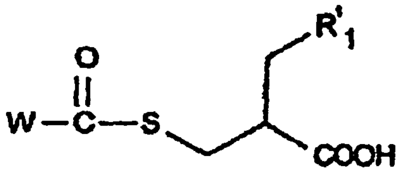

(VII)

worin $R'_1$ und W die oben angegebenen Bedeutungen haben, zu erhalten, das man: entweder der Einwirkung in Gegenwart eines Kupplungsmittels eines Produkts der Formel (VIII):

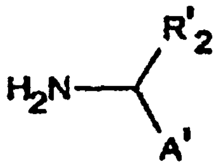

(VIII)

worin $R'_2$ die in Anspruch 1 für $R_2$ angegebene Bedeutung hat, worin die eventuellen reaktiven Funktionen gegebenenfalls geschützt sind, und A' die in Anspruch 1 für A angegebene Bedeutung hat, worin die eventuellen reaktiven Funktionen gegebenenfalls geschützt sind, unterzieht, um das Produkt der Formel (IX):

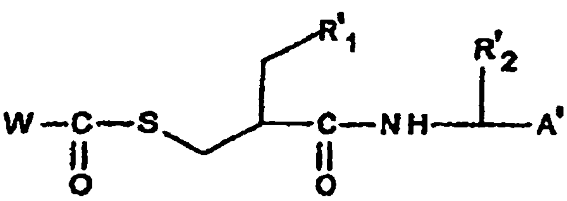

(IX)

worin $R'_1$, $R'_2$, W und A' die oben angegebenen Bedeutungen haben, zu erhalten, oder aber der Einwirkung eines Chlorierungsmittels unterzieht, um das entsprechende Säurechlorid zu erhalten, das man mit der Verbindung der Formel (VIII), wie oben definiert, umsetzt, um das Produkt der Formel (IX), wie oben definiert, zu erhalten,
oder dass man ein Produkt der Formel (X):

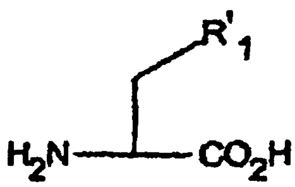

(X)

worin $R'_1$ die oben angegebene Bedeutung hat, einer Diazotierungsreaktion in Gegenwart eines Halogenierungsmittels unterzieht, um das Produkt der Formel (XI):

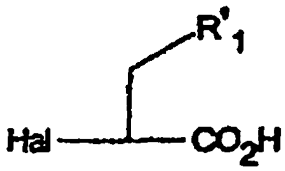

(XI)

worin R'$_1$ die oben angegebene Bedeutung hat und Hal ein Halogenatom darstellt, zu erhalten, das man der Einwirkung des Thioacetats der Formel (XIIb):

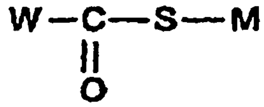

(XIIb)

worin M ein Alkalimetall darstellt und W die oben angegebene Bedeutung hat, unterzieht, um das Produkt der Formel (XIII):

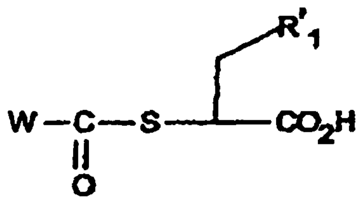

(XIII)

worin W und R'$_1$ die oben angegebenen Bedeutungen haben, zu erhalten, das man der Einwirkung des Produkts der Formel (VIII), wie oben definiert, unterzieht, um das Produkt der Formel (XIV):

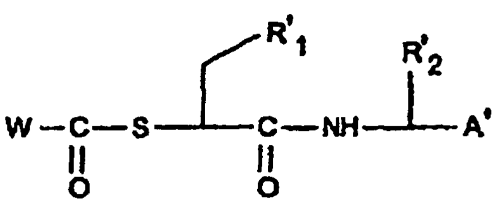

(XIV)

worin R'$_1$, R'$_2$, A' und W die oben angegebenen Bedeutungen haben, zu erhalten, Produkte der Formeln (IX) und (XIV), die man, um Produkte der Formel (I) zu erhalten oder um Produkte der Formel (I) in andere Produkte der Formel (I) umzuwandeln, wenn notwendig und wenn gewünscht, mit einer oder mehreren der folgenden Reaktionen in einer beliebigen Reihenfolge behandeln kann:

- einer Reaktion zur Verseifung einer Esterfunktion zu einer Säurefunktion,
- einer Reaktion zur Umwandlung einer Cyanofunktion in eine Säure- oder Tetrazolylfunktion,
- einer Reaktion zur Umwandlung einer Alkoxyfunktion in eine Hydroxylfunktion,
- einer Reaktion zur Ester-, Salz- oder Amidbildung einer Säurefunktion,
- einer Reaktion zur Freisetzung der Thiolfunktion aus dem Rest

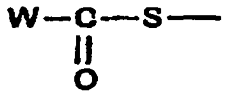

- einer Reaktion zur Entfernung der Schutzgruppen, die die geschützten reaktiven Funktionen tragen können,
- einer Reaktion zur Salzbildung mit einer anorganischen oder organischen Säure oder Base, um das entsprechende Salz zu erhalten,

wobei besagte so erhaltene Produkte der Formel (I) in allen möglichen racemischen, enantiomeren und diastereoisomeren Isomerformen vorkommen.

**7.** Die folgenden Produkte:

- N-[3-(3-Bromphenyl)-2-(mercaptomethyl)-1-oxopropyl]-L-Tryptophan,
- N-[3-((1,1'-Biphenyl)-3-yl)-2-(mercaptomethyl)-1-oxopropyl]-L-Tryptophan,
- (R,S)-N-[3-(4-Bromphenyl)-2-(mercaptomethyl)-1-oxopropyl]-L-Tryptophan,
- (R,S)-N-[3-((1,1'-Biphenyl)-4-yl)-2-(mercaptomethyl)-1-oxopropyl]-L-Tryptophan,
- (S)-N-[2-(Mercaptomethyl)-1-oxo-3-phenylpropyl]-4-(2-thienyl)-L-Phenylalanin,
- (S)-N-[2-(Mercaptomethyl)-1-oxo-3-phenylpropyl]-4-(1-naphthyl)-L-Phenylalanin,
- (S)-4-Phenyl-N-[2-(mercaptomethyl)-1-oxo-3-phenylpropyl]-L-Phenylalanin,
- (S,S)-N-(2-((1,1'-Biphenyl)-4-yl)-1-(1H-tetrazol-5-yl)-ethyl)-alpha-(mercaptomethyl)-benzenpropanamid,
- (R)- und (S)-N-[3-(3-Bromphenyl)-2-mercaptomethyl)-1-oxopropyl]-4-(2-thienyl)-L-Phenylalanin (Isomere A und B),
- N-[3-(3-Bromphenyl)-2-(mercaptomethyl)-1-oxopropyl]-7-(phenylmethoxy)-Tryptophan,

wobei besagte Produkte in allen möglichen racemischen, enantiomeren und diastereoisomeren Isomerformen vorkommen, sowie die Additionssalze mit den pharmazeutisch annehmbaren anorganischen und organischen Säuren oder mit den anorganischen oder organischen Basen besagter Produkte der Formel (I).

**8.** Die pharmazeutischen Zusammensetzungen, die als Wirkstoff wenigstens eines der Produkte, wie in Anspruch 7 definiert, enthalten.

## Claims

**1.** Use, for preparing medicinal products intended for the treatment of pathologies requiring inhibition of endothelin-converting enzyme, these pathologies being vascular spasms, vasospasm subsequent to cerebral haemorrhage, coronary spasms; peripheral vascular spasms, renal failure, myocardial infarction, congestive heart failure, atherosclerosis, pulmonary hypertension, asthma, osteoporosis, prostatic hypertrophy, and endothelin-induced hypertension, and also for preventing post-angioplasty restenosis and cardiac and vascular fibrosis, of products of formula (I):

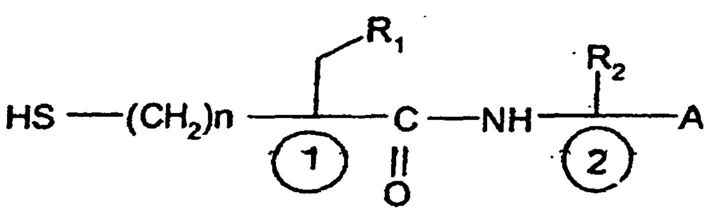

(I)

in which:

n represents the integer 0 or 1,
$R_1$ represents a phenyl or biphenyl radical optionally substituted with one or more radicals chosen from halogen atoms, and the following radicals: optionally protected hydroxyl, linear or branched alkoxy containing at most 4 carbon atoms, phenoxy, cyano, free, salified, esterified or amidated carboxyl, benzyloxy, and the dioxol radical,
$R_2$ represents a methyl radical substituted with' a phenyl, phenylthio or indolyl radical and optionally with a second phenyl radical, these phenyl, phenylthio and indolyl radicals being optionally substituted with one or more radicals chosen from halogen atoms, ahd the following radicals: optionally protected hydroxyl, linear or branched alkoxy containing at most 4 carbon atoms, cyano, free, salified, esterified or amidated carboxyl,

benzyloxy, thienyl, naphthyl and phenyl, the latter three radicals being themselves optionally substituted with one or more radicals chosen from halogen atoms, and the following radicals: optionally protected hydroxyl, linear or branched alkoxy containing at most 4 carbon atoms, cyano and free, salified, esterified or amidated carboxyl,

A represents the free, salified, esterified or amidated carboxyl radical, the free or salified tetrazolyl radical, or an alkyl radical, containing at most 10 carbon atoms and substituted with a radical chosen from free, salified, esterified or amidated carboxyl radicals, and the following radicals: optionally protected hydroxyl, alkoxy containing at most 4 carbon atoms, phenoxy, phenyl, naphthyl, thienyl, indolyl and pyridyl, these radicals being optionally substituted with one or more radicals chosen from halogen atoms, and the following radicals: optionally protected hydroxyl, linear or branched alkoxy containing at most 4 carbon atoms, cyano and free, salified, esterified or amidated carboxyl,

(1) and (2) indicating, where appropriate, the asymmetric centres of the products of formula (I), said products of formula (I) being in all the possible racemic, enantiomeric and diastereoisomeric isomer forms, and also the addition salts with mineral and organic acids or with mineral and organic bases of said products of formula (I), with the exclusion of thiorphan.

**2.** Use as defined in Claim 1, in which:

n represents the integer 0 or 1,

$R_1$ represents a phenyl or biphenyl radical optionally substituted with one or two radicals chosen from halogen atoms, and the following radicals: optionally protected hydroxyl, linear or branched alkoxy containing at most 4 carbon atoms, benzyloxy, and the dioxol radical,

$R_2$ represents a methyl radical substituted with a phenyl, phenylthio or indolyl radical and optionally with a second phenyl radical, these phenyl, phenylthio and indolyl radicals being optionally substituted with a radical chosen from the following radicals: optionally protected hydroxyl, linear or branched alkoxy containing at most 4 carbon atoms, benzyloxy, thienyl, naphthyl and phenyl, itself optionally substituted with an optionally protected hydroxyl radical or a linear or branched alkoxy radical containing at most 4 carbon atoms,

and A represents the free, salified, esterified or amidated carboxyl radical, the free or salified tetrazolyl radical, or an alkyl radical, containing at most 8 carbon atoms and substituted with a radical chosen from free, salified, esterified or amidated radicals, and the following radicals: optionally protected hydroxyl, alkoxy containing at most 4 carbon atoms, and phenoxy,

said products of formula (I) being in all the possible racemic, enantiomeric and diastereoisomeric isomer forms, and also the addition salts with mineral and organic acids or with mineral and organic bases of said products of formula (I).

**3.** Use as defined in Claim 1 or 2, in which n, $R_2$ and A have the meanings indicated in Claim 1 or 2,
and $R_1$ represents a phenyl or biphenyl radical optionally substituted either with a bromine atom or a benzyloxy radical, or with a dioxol radical and optionally a halogen atom,
said products of formula (I) being in all the possible racemic, enantiomeric and diastereoisomeric isomer forms, and also the addition salts with mineral and organic acids or with mineral and organic bases of said products of formula (I).

**4.** Use as defined in Claims 1 to 3, in which:

n represents the integer 1,

$R_1$ represents a phenyl or biphenyl radical optionally substituted either with a bromine atom or a benzyloxy radical or with a dioxol radical and optionally a halogen atom,

$R_2$ represents a methyl radical substituted either with an indolyl radical, itself optionally substituted with a benzyloxy radical or with two phenyl radicals, or with a phenylthio or phenyl radical, itself optionally substituted with a thienyl, naphthyl or phenyl radical, itself optionally substituted with an optionally protected hydroxyl radical, an alkoxy radical containing at most 4 carbon atoms or a benzyloxy radical,

A represents the free, salified, esterified or amidated carboxyl radical or an alkyl radical containing at most 8 carbon atoms, substituted with a phenoxy radical, said products of formula (I) being in all the possible racemic, enantiomeric and diastereoisomeric isomer forms, and also the addition salts with mineral and organic acids or with mineral and organic bases of said products of formula (I).

**5.** Use as defined in Claim 1, of the products having the following names:

- N-[3-(3-bromophenyl)-2-(mercaptomethyl)-1-oxopropyl]-L-tryptophan,
- N-(3-((1,1'-biphenyl)-3-yl)-2-(mercaptomethyl)-1-oxopropyl]-L-tryptophan,
- (R,S)-N-(3-(4-bromophenyl)-2-(mercaptomethyl)-1-oxopropyl]-L-tryptophan,
- (R,S)-N-(3-((1,1'-biphenyl)-4-yl)-2-(mercaptomethyl)-1-oxopropyl]-L-tryptophan,
- (S)-N-[2-(mercaptomethyl)-1-oxo-3-phenylpropyl]-4-(2-thienyl)-L-phenylalanine,
- (S)-N-[2-(mercaptomethyl)-1-oxo-3-phenylpropyl]-4-(1-naphthyl)-L-phenylalanine,
- (S)-N-[2-(mercaptomethyl)-1-oxo-3-phenylpropyl]-L-tryptophan,
- (S)-4-phenyl-N-[(2-mercaptomethyl)-1-oxo-3-phenylpropyl]-L-phenylalanine,
- (S,S)-N-(2-((1,1'-biphenyl)-4-yl)-1-(1H-tetrazol-5-yl)-ethyl)-alpha-(mercaptomethyl)benzenepropanamide,
- (R) and (S)-N-[3-(3-bromophenyl)-2-(mercaptomethyl)-1-oxopropyl]-4-(2-thienyl)-L-phenylalanine (isomers A and B),
- N-[3-(3-bromophenyl)-2-(mercaptomethyl)-1-oxopropyl]-7-(phenylmethoxy)tryptophan,

said products of formula (I) being in all the possible racemic, enantiomeric and diastereoisomeric isomer forms, and also the addition salts with mineral and organic acids or with mineral and organic bases of said products of formula (I).

6. Method for preparing the products of formula (I) for the use as defined in Claim 1, **characterized in that** **either** a product of formula (II):

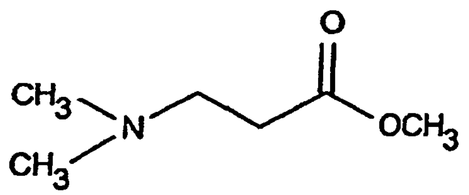

(II)

is subjected to the action of a product of formula (III):

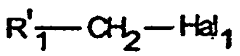

(III)

in which $Hal_1$ represents a chlorine or iodine atom, and $R'_1$ has the meaning indicated in Claim 1 for $R_1$ in which the possible reactive functions are optionally protected, so as to obtain the product of formula (IV):

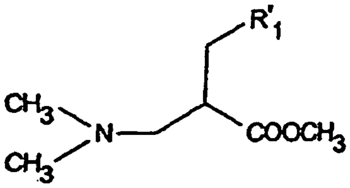

(IV)

in which $R'_1$ has the meaning indicated above,
which is subjected to the action of methyl iodide so as to obtain the product of formula (V):

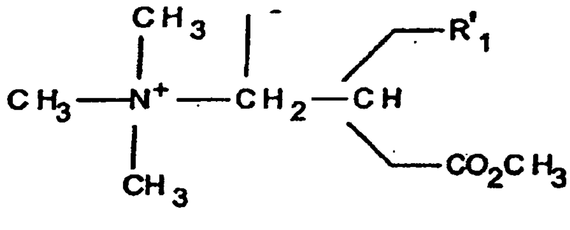

(V)

in which $R'_1$ has the meaning indicated above, which is subjected to an elimination and saponification reaction, so as to obtain the product of formula (VI):

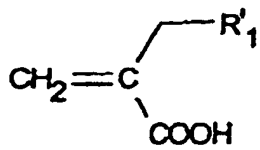

(VI)

in which $R'_1$ has the meaning indicated above for $R_1$, which is subjected to the action of the compound of formula (XIIa):

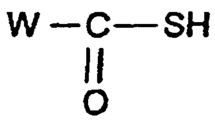

(XIIa)

in which W represents an alkyl or phenyl radical, so as to obtain the product of formula (VII):

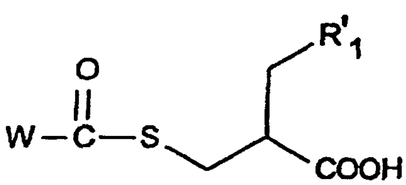

(VII)

in which $R'_1$ and W have the meanings indicated above, which is subjected:

either to the action, in the presence of a coupling agent, of a product of formula (VIII):

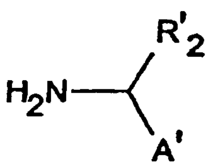

(VIII)

in which $R'_2$ has the meaning indicated in Claim 1 for $R_2$ in which the possible reactive functions are optionally protected, and A' has the meaning indicated in Claim 1 for A, in which the possible reactive functions are optionally protected, so as to obtain the product of formula (IX):

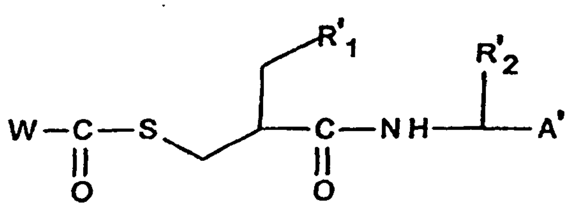

(IX)

in which $R'_1$, $R'_2$, W and A' have the meanings indicated above,
or else to the action of a chlorinating agent so as to obtain the corresponding acid chloride, which is reacted with

the compound of formula (VIII) as defined above, so as to obtain the product of formula (IX) as defined above, or a product of formula (X):

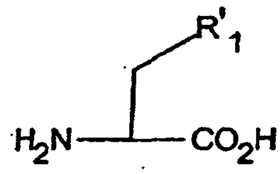

(X)

in which R'$_1$ has the meaning indicated above, is subjected to a diazotation reaction in the presence of a halogenating agent, so as to obtain the product of formula (XI):

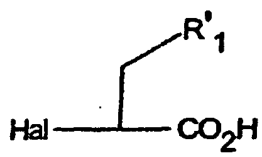

(XI)

in which R'$_1$ has the meaning indicated above and Hal represents a halogen atom, which product is subjected to the action of the thioacetate of formula (XIIb):

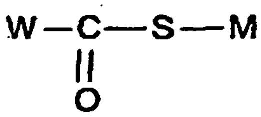

(XIIb)

in which M represents an alkali metal and W has the meaning indicated above, so as to obtain the product of formula (XIII):

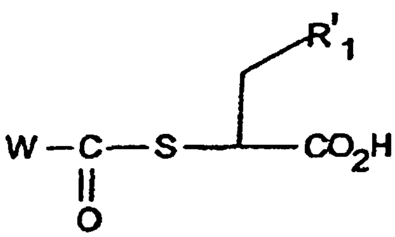

(XIII)

in which W and R'$_1$ have the meanings indicated above, which product is subjected to the action of the product of formula (VIII) as defined above, so as to obtain the product of formula (XIV):

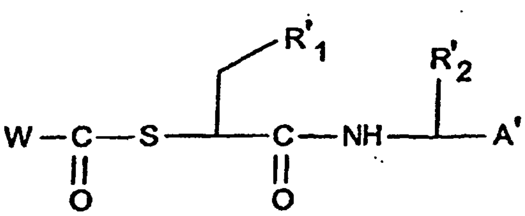

(XIV)

in which R'$_1$, R'$_2$, A' and W have the meanings indicated above,
which products of formulae (IX) and (XIV) can, in order to obtain products of formula (I) or to convert products of

formula (I) to other products of formula (I), be treated, if desired and if necessary, with one or more of the following reactions, in any order:

- a reaction consisting of saponification of an ester function to an acid function,
- a reaction consisting of conversion of a cyano function to an acid or tetrazolyl function,
- a reaction consisting of conversion of an alkoxy function to a hydroxyl function,
- a reaction consisting of esterification, salification or amidation of an acid function,
- a reaction consisting of release of the thiol function from the radical

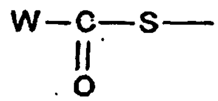

- a reaction consisting in elimination of the protective groups that the protected reactive functions may carry,
- a reaction consisting of salification with a mineral or organic acid or base, so as to obtain the corresponding salt,

said products of formula (I) thus obtained being in all the possible racemic, enantiomeric and diastereoisomeric isomer forms.

**7.** The following products:

- N-[3-(3-bromophenyl)-2-(mercaptomethyl)-1-oxopropyl]-L-tryptophan,
- N-(3-((1,1'-biphenyl)-3-yl)-2-(mercaptomethyl)-1-oxopropyl]-L-tryptophan,
- (R,S)-N-(3-(4-bromophenyl)-2-(mercaptomethyl)-1-oxopropyl]-L-tryptophan,
- (R,S)-N-(3-((1,1'-biphenyl)-4-yl)-2-(mercaptomethyl)-1-oxopropyl]-L-tryptophan,
- (S) -N-[2- (mercaptomethyl) -1-oxo-3-phenylpropyl] -4-(2-thienyl)-L-phenylalanine,
- (S)-N-[2-(mercaptomethyl)-1-oxo-3-phenylpropyl]-4-(1-naphthyl)-L-phenylalanine,
- (S)-4-phenyl-N-((2-mercaptomethyl)-1-oxo-3-phenylpropyl)-L-phenylalanine,
- (S,S)-N-(2-((1,1'-biphenyl)-4-yl)-1-(1H-tetrazol-5-yl)-ethyl)-alpha-(mercaptomethyl)benzenepropanamide,
- (R) and (S)-N-(3-(3-bromophenyl)-2-(mercaptomethyl)-1-oxopropyl]-4-(2-thienyl)-L-phenylalanine (isomers A and B),
- N-[3-(3-bromophenyl)-2-(mercaptomethyl)-1-oxopropyl]-7-(phenylmethoxy)tryptophan,

said products being in all the possible racemic, enantiomeric and diastereoisomeric isomer forms, and also the addition salts with mineral and organic acids or with mineral and organic bases, that are pharmaceutically acceptable, of said products of formula (I).

**8.** Pharmaceutical compositions containing, as active principle, at least one of the products as defined in Claim 7.